# FASCICULE DE BREVET EUROPEEN

(11) **EP 0 755 449 B1**
(45) Date de publication et mention de la délivrance du brevet: **24.11.2004**
(21) Numéro de dépôt: 95917378.2
(22) Date de dépôt: 11.04.1995
(51) Int. Cl.: C12N 15/53, C12N 15/11, C12N 15/82, C12N 9/02, A01H 5/00

(54) **SEQUENCES D'ADN CODANT POUR UNE CINNAMOYL CoA REDUCTASE, ET LEURS APPLICATIONS DANS LE DOMAINE DE LA REGULATION DES TENEURS EN LIGNINES DES PLANTES**
FÜR EINE CINNAMOYL-COA-REDUKTASE KODIERENDE DNA-SEQUENZEN, UND IHRE VERWENDUNG IN REGULATION DES LIGNIN-GEHALTS VON PFLANZEN
DNA SEQUENCES CODING FOR A CYNNAMOYL CoA REDUCTASE AND THEIR USE IN THE REGULATION OF PLANT LIGNIN CONCENTRATIONS

(30) Priorité: 11.04.1994 FR 9404246
(43) Date de publication de la demande: 29.01.1997
(73) Titulaire: Biogemma, 75001 Paris (FR)
(72) Inventeur: BOUDET, Alain, F-31400 Toulouse (FR); PETTENATI, Jacqueline, F-31450 Fourqueveaux (FR); GOFFNER, Deborah, F-31320 Vieille Toulouse (FR); ROMESTANT, Michel, 12000 Rodez (FR); LEPLE, Jean-Charles, 45150 Ferolles (FR); O'CONNELL, Ann, Weybridge, Surrey KT13 9YR (GB); BOERJAN, Wout, 9270 Kalken (BE); HALPIN, Claire, Fife DD8 8RG (GB)
(74) Mandataire: Flesselles, Bruno F.G.
(86) Numéro de dépôt international: PCT/FR1995/000465
(87) Numéro de publication internationale: WO 1995/027790

(56) Documents cités:
- EP-A- 0 155 872
- WO-A-93/05159
- THEORETICAL AND APPLIED GENETICS, vol. 87, no. 8, Mars 1994 pages 1006-1015, CARRON, T.R., ET AL. 'GENETIC MODIFICATION OF CONDENSED TANNIN BIOSYNTHESIS IN LOTUS CORNICULATUS.1. HETEROLOGOUS ANTISENSE DIHYDROFLAVONOL REDUCTASE DOWN-REGULATES TANNIN ACCUMULATION IN "HAIRY ROOT" CULTURES'
- BULL. LIASON - GROUPE POLYPHENOLS, vol. 16(PT. 2), 1992 pages 295-300, ROBBINS, M.P., ET AL. 'MANIPULATION OF CONDENSED TANNIN BIOSYNTHESIS IN FORAGE LEGUMES'
- J. CELL. BIOCHEM. SUPPL., vol. 17A, 1993 page 26 CAMPBELL, M.M., ET AL. 'Hydroxycinnamoyl-coA reductase from Eucalyptus. Molecular analysis of a key control point of lignification' & KEYSTONE SYMPOSIUM ON THE EXTRACELLULAR MATRIX OF PLANTS: MOLECULAR, CELLUALR AND DEVELOPMENTAL BIOLOGY, SANT FE, NEW MEXICO, USA, JANUARY 9-15, 1993.,
- EUR. J. BIOCHEM., vol. 139, 1984 pages 259-265, SARNI, F., ET AL. 'PURIFICATION AND PROPERTIES OF CINNAMOYL-COA-REDUCTASE AND CIMMAMYL ALCOHOL DEHYDROGENASE FROM POPLAR STEMS(POPULUS X UERAMERICAN)'
- EMBL ACCESSION NO. T13840, REL. 38., 7-MAR-1994, 2005 ARABIDOPSIS THALIANA CDNA CLONE 41H9T7
- NEW PHYTOLOGIST 129 (2). 1995. 203-236., BOUDET A M ET AL 'Tansley review no. 80: Biochemistry and molecular biology of lignification.'
- PLANT PHYSIOLOGY (ROCKVILLE) 106 (2).OCTOBRE 1994. 625-632. , GOFFNER D ET AL 'Purification and characterization of cinnamoyl-coenzyme A:NADP oxidoreductase in Eucalyptus gunnii.'
- PLANT MOLECULAR BIOLOGY, vol. 13, 1989 pages 491-502, BELD, M., ET AL 'FLAVONOID SYNTHESIS IN PETUNIA HYBRIDA: PARTIAL CHARACTERIZATION OF DIHYDROFLAVONOL-4-REDUCTASE GENES'

## Description

La présente invention a pour objet l'utilisation de séquences d'ADN codant pour une cinnamoyl CoA réductase (CCR) chez les plantes, ou de tout fragment de ces séquences, ou encore de toute séquence dérivée de ces dernières, ou de leurs séquences complémentaires, dans le cadre de la mise en oeuvre de procédés de régulation du taux de lignine chez les plantes.

La lignine est un polymère aromatique hétérogène complexe qui imperméabilise et renforce les parois de certaines cellules des plantes.

La lignine est formée par polymérisation de radicaux libres dérivant de monolignols tels que les alcools paracoumarylique, coniférylique et sinapylique (Higuchi, 1985, in Biosynthesis and degradation of wood components (T. Higuchi, ed), Academic Press, Orlando, FL, pp. 141-160).

Les lignines présentent une grande variation dans leur contenu relatif en monolignols, en fonction des espèces, et des différents tissus au sein d'une même plante.

Cette variation est probablement due et contrôlée par les différentes activités et spécificités de substrats, des enzymes nécessaires à la biosynthèse des monomères de la lignine (Higuchi, 1985, susmentionné).

Au-delà de son rôle dans la structure et le développement des plantes, la lignine représente un composant majeur de la biomasse terrestre et revêt une grande importance économique et écologique (Brown, 1985, J. Appl. Biochem. 7, 371-387; Whetten and Sederoff, 1991, Forest Ecology and Management, **43**, 301-316).

Sur le plan de l'exploitation de la biomasse, il convient tout d'abord de noter que la lignine est un facteur limitant de la digestibilité et du rendement nutritionnel des plantes fourragères. En effet, il est clairement démontré que la digestibilité des plantes fourragères par les ruminants, est inversement proportionnelle à la teneur en lignines de ces plantes. La nature des lignines étant également un facteur déterminant dans ce phénomène (Buxton and Roussel, 1988, Crop. Sci., **28,** 553-558; Jung and Vogel, 1986, J. Anim., Sci., **62,** 1703-1712).

Parmi les principales plantes fourragères chez lesquelles il serait intéressant de diminuer les teneurs en lignines, on peut citer: luzerne, fétuque, maïs, fourrage utilisé en ensilage...

Notons également que des teneurs en lignines élevées sont en partie responsables de la qualité limitée des tourteaux de tournesol destinés à l'alimentation du bétail, et de la diminution des capacités germinatives de certaines semences dans le domaine de l'horticulture.

On peut souligner également que la lignification intense qui se produit lors de la conservation des organes végétaux après récolte, rend rapidement impropres à la consommation, des productions telles que l'asperge, l'igname, les carottes, etc...

Par ailleurs, il convient de noter également que plus de 50 millions de tonnes de lignines sont extraites de la matière ligneuse chaque année dans le cadre de la production de la pâte à papier dans l'industrie papetière. Cette opération d'extraction nécessaire à l'obtention de la cellulose est énergétiquement coûteuse et secondairement polluante à travers les composés chimiques mis en jeu pour l'extraction et que l'on retrouve dans l'environnement (Dean and Eriksson, 1992, Holzforschung, **46**, 135-147; Whetten and Sederoff, 1991, susmentionné).

Réduire les proportions en lignines (qui selon les espèces, représentent de 20 à 30% de la matière sèche) de quelques pour cents (2 à 5%), représenterait un gain de rendement, une économie substantielle (produits chimiques) et contribuerait à l'amélioration de l'environnement (réduction des pollutions). Etant donné les échelles d'utilisation de la matière ligneuse, ces retombées auraient des répercussions extrêmement importantes. Dans ce cas, les espèces concernées pourraient être le peuplier, l'eucalyptus, l'*Acacia mangium*, le genre *Casuarina* et l'ensemble des angiospermes et gymnospermes utilisés pour la production de pâte à papier.

Il est clair que, dans les deux domaines considérés, la réduction des taux de lignines doit être modérée pour conserver à la plante (ou à l'arbre) ses caractéristiques de rigidité et son architecture normale puisque les lignines qui consolident les parois cellulaires jouent un rôle important dans le maintien du port dressé des végétaux.

Les variations naturelles dans les teneurs en lignines observées dans la nature pour une même espèce (écarts pouvant aller jusqu'à 6-8% de la masse sèche entre individus) autorisent les diminutions évoquées plus haut.

La résistance à la dégradation de la lignine, de même que les difficultés que l'on rencontre dans le cadre de son extraction, sont probablement dues à la structure complexe de ce polymère constitué de liaisons éther et carbone-carbone entre les monomères, ainsi qu'aux nombreuses liaisons chimiques existant entre la lignine et d'autres composants de la paroi cellulaire (Sarkanen and Ludwig, 1971, in Lignins: Occurrence, Formation, Structure and Reactions (K.V. Sarkanen and C.H. Kudwig eds) New York: Wiley - Interscience, pp. 1-18).

Partant des cinnamoyls-CoA, la biosynthèse des lignines chez les plantes, est effectuée de la manière suivante:

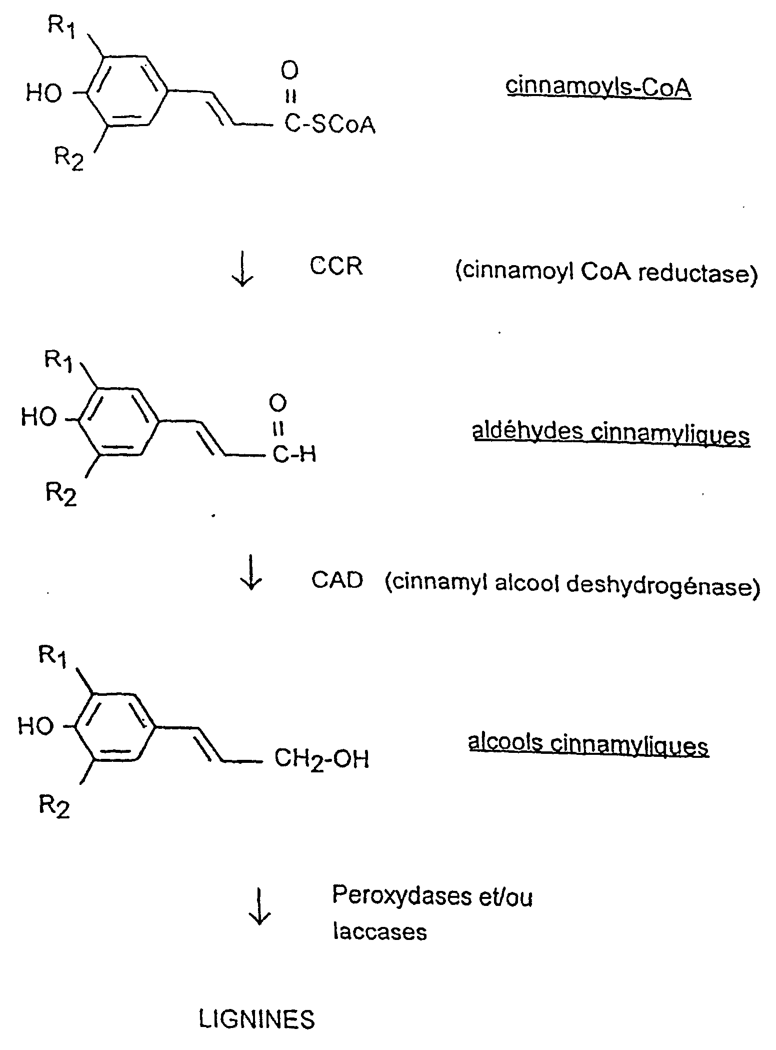

Une approche, par voie de génie génétique, pour tenter de réduire le taux de lignines chez les plantes, consisterait à inhiber la synthèse d'une des enzymes de la chaîne de la biosynthèse de ces lignines indiquées ci-dessus.

Une technique particulièrement appropriée dans le cadre d'une telle approche, est celle de l'utilisation d'ARNm antisens susceptible de s'hybrider avec l'ARNm codant pour ces enzymes, et par conséquent, d'empêcher, pour le moins partiellement, la production de ces enzymes à partir de leur ARNm correspondant.

Un telle stratégie antisens, réalisée à l'aide du gène codant pour la CAD chez le tabac, a fait l'objet de la demande de brevet européen n° 584 117, décrivant l'utilisation d'ARNm antisens susceptible d'inhiber la production de lignines chez les plantes en s'hybridant à l'ARNm codant pour la CAD chez ces plantes.

Les résultats au niveau des plantes ainsi transformées démontrent une réduction de l'activité de la CAD, mais paradoxalement, les teneurs en lignines ne montrent pas d'évolution. Des études complémentaires indiquent que les lignines de plantes transformées sont différentes des lignines témoins, car les aldéhydes cinnamyliques sont directement incorporés dans le polymère lignine.

L'un des buts de la présente invention est précisément celui de fournir un procédé permettant de réguler efficacement les teneurs en lignines dans les plantes, soit dans le sens d'une diminution sensible de ces teneurs par rapport aux teneurs normales dans les plantes, soit dans le sens d'une augmentation de ces teneurs.

Un autre but de la présente invention est de fournir les outils pour la mise en oeuvre d'un tel procédé, et plus particulièrement des constructions utilisables pour la transformation de plantes.

Un autre but de la présente invention est de fournir des plantes transformées génétiquement, notamment des plantes fourragères susceptibles d'être mieux digérées que les plantes non transformées, ou encore des plantes ou arbres transformés pour la production de la pâte à papier, et dont l'extraction des lignines serait facilitée et moins polluante que dans le cas d'arbres non transformés.

Un autre but de la présente invention est celui de fournir des plantes transformées davantage résistantes à des attaques de l'environnement, notamment à des attaques parasitaires, que ne le sont les plantes non transformées, ou encore des plantes transformées de plus grande taille, ou de taille plus réduite (que celle des plantes non transformées).

La présente invention a pour objet l'utilisation de séquences nucléotidiques recombinantes contenant une (ou plusieurs) région(s) codante(s), cette (ces) région(s) codante(s) étant constituée(s) :
- d'une séquence nucléotidique codant pour un ARN messager (ARNm), cet ARNm codant lui-même pour une cinnamoyl CoA réductase (CCR) chez les plantes, ou d'un fragment de la séquence nucléotidique susmentionnée, ce fragment codant pour un ARNm, cet ARNm codant lui-même pour un fragment d'une CCR chez les plantes, ce fragment de CCR présentant une activité enzymatique équivalente à celle de la CCR susmentionnée, ou d'une séquence nucléotidique dérivée de la séquence nucléotidique susmentionnée, ou dérivée du fragment susmentionné, notamment par mutation et/ou addition, et/ou suppression, et/ou substitution d'un ou plusieurs nucléotides, cette séquence dérivée codant pour un ARNm, cet ARNm codant lui-même pour une protéine dérivée présentant une activité enzymatique équivalente à celle de la CCR susmentionnée, ou
- d'une séquence nucléotidique complémentaire de tout ou partie de la séquence nucléotidique codant pour un ARNm, ou du fragment de cette séquence, ou de la séquence dérivée de ces derniers, tels que définis ci-dessus, cette séquence complémentaire codant pour une séquence nucléotidique antisens (encore désignée ARNm antisens) susceptible de s'hybrider avec l'un des ARNm susmentionnés,
pour la transformation de cellules végétales en vue de l'obtention de plantes transgéniques au sein desquelles la biosynthèse des lignines est régulée soit dans le sens d'une augmentation, soit dans le sens d'une diminution des teneurs en lignines produites, par rapport aux teneurs normales en lignines produites chez les plantes.

Par l'expression "séquence nucléotidique dérivée", dans ce qui précède et ce qui suit, on entend toute séquence présentant au moins environ 50% de nucléotides homologues à ceux de la séquence dont elle dérive.

Par "protéine dérivée" dans ce qui précède et ce qui suit, on entend toute protéine présentant au moins environ 50% d'acides aminés homologues à ceux de la protéine dont elle dérive.

Parmi les séquences nucléotidiques susceptibles d'être utilisées à titre de régions codantes dans les séquences nucléotidiques recombinantes, on peut citer principalement:
- la séquence nucléotidique représentée par SEQ ID NO 1, codant pour un ARNm, cet ARNm codant lui-même pour la CCR d'eucalyptus représentée par SEQ ID NO 2,
- la séquence nucléotidique représentée par SEQ ID NO 3, codant pour un ARNm, cet ARNm codant lui-même pour la CCR de peuplier représentée par SEQ ID NO 4,
- la séquence nucléotidique représentée par SEQ ID NO 5, codant pour un ARNm, cet ARNm codant lui-même pour la CCR de fétuque représentée par SEQ ID NO 6,
- la séquence nucléotidique représentée par SEQ ID NO 7, codant pour un ARNm, cet ARNm codant lui-même pour la CCR de tabac représentée par SEQ ID NO 8,
- la séquence nucléotidique complémentaire de celle représentée par SEQ ID NO 1, SEQ ID NO 3, SEQ ID NO 5 ou SEQ ID NO 7, cette séquence complémentaire codant pour un ARNm antisens susceptible de s'hybrider avec l'ARNm codé par les séquences SEQ ID NO 1, SEQ ID NO 3, SEQ ID NO 5 et SEQ ID NO 7 respectivement,
- la séquence nucléotidique dérivée de la séquence représentée par SEQ ID NO 1, SEQ ID NO 3, SEQ ID NO 5 ou SEQ ID NO 7, notamment par mutation et/ou addition, et/ou suppression, et/ou substitution d'un ou plusieurs nucléotides, cette séquence dérivée codant soit pour un ARNm codant lui-même pour la CCR représentée par SEQ ID NO 2, SEQ ID NO 4, SEQ ID NO 6 ou SEQ ID NO 8 respectivement, ou pour une protéine dérivée de ces dernières et présentant une activité enzymatique équivalente à celle desdites CCR chez les plantes, notamment la séquence nucléotidique dérivée de la séquence SEQ ID NO 1, et représentée par SEQ ID NO 9, cette dernière codant pour un ARNm, cet ARNm codant lui-même pour une protéine représentée par SEQ ID NO 10 dérivée de la CCR d'eucalyptus susmentionnée,
- la séquence nucléotidique dérivée de la séquence nucléotidique complémentaire susmentionnée, par mutation et/ou addition, et/ou suppression, et/ou substitution d'un ou plusieurs nucléotides, cette séquence dérivée codant pour un ARNm antisens susceptible de s'hybrider avec un des ARNm susmentionnés.

La présente invention a plus particulièrement pour objet toute séquence d'ADN, caractérisée en ce qu'elle comprend à titre de région codante:
- la séquence nucléotidique représentée par SEQ ID NO 1, codant pour un ARNm, cet ARNm codant lui-même pour la CCR représentée par SEQ ID NO 2, ou
- un fragment de la séquence nucléotidique susmentionnée, ce fragment codant pour un fragment de la CCR représentée par SEQ ID NO 2, ce fragment de CCR présentant une activité enzymatique équivalente à celle de la CCR susmentionnée, ou
- toute séquence nucléotidique dérivée de la séquence représentée par SEQ ID NO 1 susmentionnée, ou d'un fragment tel que décrit ci-dessus de cette séquence, notamment par mutation et/ou addition, et/ou suppression, et/ou substitution d'un ou plusieurs nucléotides, cette séquence dérivée codant pour un ARNm codant lui-même pour la CCR représentée par SEQ ID NO 2, ou pour une protéine dérivée de cette dernière et présentant une activité enzymatique équivalente à celle de ladite CCR chez les plantes, notamment la séquence nucléotidique dérivée de la séquence SEQ ID NO 1, et représentée par SEQ ID NO 9, cette dernière codant pour un ARNm, cet ARNm codant lui-même pour une protéine représentée par SEQ ID NO 10 dérivée de la CCR d'eucalyptus susmentionnée.

La présente invention a plus particulièrement pour objet toute séquence d'ADN, caractérisée en ce qu'elle comprend à titre de région codante:
- la séquence nucléotidique représentée par SEQ ID NO 3, codant pour ARNm, cet ARNm codant lui-même pour la CCR représentée par SEQ ID NO 4, ou
- un fragment de la séquence nucléotidique susmentionnée, ce fragment codant pour un fragment de la CCR représentée par SEQ ID NO 4, ce fragment de CCR présentant une activité enzymatique équivalente à celle de la CCR susmentionnée, ou
- toute séquence nucléotidique dérivée de la séquence représentée par SEQ ID NO 3, susmentionnée, ou d'un fragment tel que décrit ci-dessus de cette séquence, notamment par mutation et/ou addition, et/ou suppression, et/ou substitution d'un ou plusieurs nucléotides, cette séquence dérivée codant pour un ARNm codant lui-même pour la CCR représentée par SEQ ID NO 4, ou pour une protéine dérivée de cette dernière et présentant une activité enzymatique équivalente à celle de ladite CCR chez les plantes.

La présente invention a plus particulièrement pour objet toute séquence d'ADN, caractérisée en ce qu'elle comprend à titre de région codante:
- la séquence nucléotidique représentée par SEQ ID NO 5, codant pour ARNm, cet ARNm codant lui-même pour la CCR représentée par SEQ ID NO 6, ou
- un fragment de la séquence nucléotidique susmentionnée, ce fragment codant pour un fragment de la CCR représentée par SEQ ID NO 6, ce fragment de CCR présentant une activité enzymatique équivalente à celle de la CCR susmentionnée, ou
- toute séquence nucléotidique dérivée de la séquence représentée par SEQ ID NO 5, susmentionnée, ou d'un fragment tel que décrit ci-dessus de cette séquence, notamment par mutation et/ou addition, et/ou suppression, et/ou substitution d'un ou plusieurs nucléotides, cette séquence dérivée codant pour un ARNm codant lui-même pour la CCR représentée par SEQ ID NO 6, ou pour une protéine dérivée de cette dernière et présentant une activité enzymatique équivalente à celle de ladite CCR chez les plantes.

La présente invention a plus particulièrement pour objet toute séquence d'ADN, caractérisée en ce qu'elle comprend à titre de région codante:
- la séquence nucléotidique représentée par SEQ ID NO 7, codant pour ARNm, cet ARNm codant lui-même pour la CCR représentée par SEQ ID NO 8, ou
- un fragment de la séquence nucléotidique susmentionnée, ce fragment codant pour un fragment de la CCR représentée par SEQ ID NO 8, ce fragment de CCR présentant une activité enzymatique équivalente à celle de la CCR susmentionnée, ou
- toute séquence nucléotidique dérivée de la séquence représentée par SEQ ID NO 7, susmentionnée, ou d'un fragment tel que décrit ci-dessus de cette séquence, notamment par mutation et/ou addition, et/ou suppression, et/ou substitution d'un ou plusieurs nucléotides, cette séquence dérivée codant pour un ARNm codant lui-même pour la CCR représentée par SEQ ID NO 8, ou pour une protéine dérivée de cette dernière et présentant une activité enzymatique équivalente à celle de ladite CCR chez les plantes.

Par protéine présentant une activité enzymatique équivalente à celle des CCR présentes chez les plantes, et plus particulièrement des CCR représentées par SEQ ID NO 2, SEQ ID NO 4, SEQ ID NO 6 et SEQ ID NO 8, on entend toute protéine possédant une activité CCR telle que mesurée selon la méthode de Luderitz et Grisebach publiée dans Eur. J. Biochem. (1981), **119**: 115-127.

A titre d'illustration, cette méthode est réalisée par mesure spectrophotométrique de l'activité réductrice de la protéine (CCR ou dérivée), en suivant la disparition des cinnamoyl CoA à 366 nm. La réaction se déroule à 30°C, pendant 2 à 10 minutes. La composition du milieu réactionnel est la suivante: tampon phosphate 100 mM, pH 6.25, 0,1 mM NADPH, 70 µM Feruloyl CoA, 5 à 100 µl d'extrait enzymatique dans un volume total de 500 µl.

L'invention a également pour objet toute séquence d'ADN, caractérisée en ce qu'elle comprend à titre de région codante:
- la séquence nucléotidique complémentaire de celle représentée par SEQ ID NO 1, cette séquence complémentaire codant pour un ARNm antisens susceptible de s'hybrider avec l'ARNm codant lui-même pour la CCR représentée par SEQ ID NO 2, à savoir l'ARNm codé par la séquence représentée par SEQ ID NO 1, ou codé par une séquence dérivée de cette dernière, telle que définie ci-dessus, ou
- un fragment de la séquence complémentaire susmentionnée, ce fragment de séquence codant pour un ARNm antisens susceptible de s'hybrider avec l'ARNm codant lui-même pour la CCR représentée par SEQ ID NO 2, tel que défini ci-dessus, ou
- toute séquence nucléotidique dérivée de la séquence complémentaire susmentionnée, ou du fragment de cette séquence complémentaire tel que décrit ci-dessus, notamment par mutation et/ou addition, et/ou suppression, et/ou substitution d'un ou plusieurs nucléotides, cette séquence dérivée codant pour un ARNm antisens susceptible de s'hybrider avec l'ARNm susmentionné, notamment la séquence complémentaire de celle représentée par SEQ ID NO 9.

La présente invention a plus particulièrement pour objet toute séquence d'ADN, caractérisée en ce qu'elle comprend à titre de région codante:
- la séquence nucléotidique complémentaire de celle représentée par SEQ ID NO 3, cette séquence complémentaire codant pour un ARNm antisens susceptible de s'hybrider avec l'ARNm codant lui-même pour la CCR représentée par SEQ ID NO 4, à savoir l'ARNm codé par la séquence représentée par SEQ ID NO 3, ou codé par une séquence dérivée de cette dernière, telle que définie ci-dessus, ou
- un fragment de la séquence complémentaire susmentionnée, ce fragment de séquence codant pour un ARNm antisens susceptible de s'hybrider avec l'ARNm codant lui-même pour la CCR représentée par SEQ ID NO 4, tel que défini ci-dessus, ou
- toute séquence nucléotidique dérivée de la séquence complémentaire susmentionnée, ou du fragment de cette séquence complémentaire tel que décrit ci-dessus, notamment par mutation et/ou addition, et/ou suppression, et/ou substitution d'un ou plusieurs nucléotides, cette séquence dérivée codant pour un ARNm antisens susceptible de s'hybrider avec l'ARNm susmentionné.

La présente invention a plus particulièrement pour objet toute séquence d'ADN, caractérisée en ce qu'elle comprend à titre de région codante:
- la séquence nucléotidique complémentaire de celle représentée par SEQ ID NO 5, cette séquence complémentaire codant pour un ARNm antisens susceptible de s'hybrider avec l'ARNm codant lui-même pour la CCR représentée par SEQ ID NO 6, à savoir l'ARNm codé par la séquence représentée par SEQ ID NO. 5, ou codé par une séquence dérivée de cette dernière, telle que définie ci-dessus, ou
- un fragment de la séquence complémentaire susmentionnée, ce fragment de séquence codant pour un ARNm antisens susceptible de s'hybrider avec l'ARNm codant lui-même pour la CCR représentée par SEQ ID NO 5, tel que défini ci-dessus, ou
- toute séquence nucléotidique dérivée de la séquence complémentaire susmentionnée, ou du fragment de cette séquence complémentaire tel que décrit ci-dessus, notamment par mutation et/ou addition, et/ou suppression, et/ou substitution d'un ou plusieurs nucléotides, cette séquence dérivée codant pour un ARNm antisens susceptible de s'hybrider avec l'ARNm susmentionné.

La présente invention a plus particulièrement pour objet toute séquence d'ADN, caractérisée en ce qu'elle comprend à titre de région codante:
- la séquence nucléotidique complémentaire de celle représentée par SEQ ID NO 7, cette séquence complémentaire codant pour un ARNm antisens susceptible de s'hybrider avec l'ARNm codant lui-même pour la CCR représentée par SEQ ID NO 8, à savoir l'ARNm codé par la séquence représentée par SEQ ID NO 7, ou codé par une séquence dérivée de cette dernière, telle que définie ci-dessus, ou
- un fragment de la séquence complémentaire susmentionnée, ce fragment de séquence codant pour un ARNm antisens susceptible de s'hybrider avec l'ARNm codant lui-même pour la CCR représentée par SEQ ID NO 7, tel que défini ci-dessus, ou
- toute séquence nucléotidique dérivée de la séquence complémentaire susmentionnée, ou du fragment de cette séquence complémentaire tel que décrit ci-dessus, notamment par mutation et/ou addition, et/ou suppression, et/ou substitution d'un ou plusieurs nucléotides, cette séquence dérivée codant pour un ARNm antisens susceptible de s'hybrider avec l'ARNm susmentionné.

Il va de soi que les séquences représentées par SEQ ID NO 1, SEQ ID NO 3, SEQ ID NO 5, SEQ ID NO 7 et SEQ ID NO 9, les séquences complémentaires, les séquences dérivées et les fragments de séquences de l'invention mentionnées ci-dessus, doivent être pris en considération comme étant représentées dans le sens 5' → 3'.

Ainsi, le premier nucléotide d'une séquence complémentaire dans le sens 5' → 3' telle que décrite ci-dessus, est le complément du dernier nucléotide de la séquence dans le sens 5' → 3' codant pour une CCR (ou fragment de CCR ou protéine dérivée), le second nucléotide de cette séquence complémentaire est le complément de l'avant-dernier nucléotide de la séquence codant pour une CCR, et ainsi de suite, jusqu'au dernier nucléotide de ladite séquence complémentaire qui est le complément du premier nucléotide de la séquence codant pour une CCR.

L'ARNm codé par la séquence complémentaire susmentionnée est tel que, lorsque cet ARNm est représenté dans le sens 5' → 3', son premier nucléotide correspond au dernier nucléotide de la séquence codant pour une CCR, et donc s'hybride avec le dernier nucléotide de l'ARNm codé par cette dernière, tandis que son dernier nucléotide correspond au premier nucléotide de la séquence codant pour une CCR, et donc s'hybride avec le premier nucléotide de l'ARNm codé par cette dernière.

Ainsi, on entend par ARNm antisens dans ce qui précède et ce qui suit, tout ARNm codé par la susdite séquence complémentaire et représenté dans le sens inverse (3' → 5') du sens dans lequel est représenté l'ARNm codé par la séquence codant pour une CCR (ou fragment de CCR ou protéine dérivée), ce dernier ARNm étant encore désigné ARNm sens (5' → 3').

Le terme d'ARN antisens s'adresse donc à une séquence d'ARN complémentaire de la séquence en bases de l'ARN messager, le terme complémentaire devant être compris en ce sens que chaque base (ou une majorité de bases) de la séquence antisens (lue dans le sens 3' → 5') est capable de s'apparier avec les bases correspondantes (G avec C, A avec U) de l'ARN messager (séquence lue dans le sens 5' → 3').

La stratégie des ARN antisens, dans le cadre de la présente invention, est une approche moléculaire particulièrement adaptée à l'objectif d'une modulation des taux de lignines des plantes. L'ARN antisens est un ARN produit par la transcription du brin d'ADN non codant (brin non-sens).

Cette stratégie antisens est plus particulièrement décrite dans le brevet européen n° 240 208.

On pense que l'inhibition de la synthèse d'une protéine selon la stratégie antisens, en l'occurrence de la CCR dans le cas présent, est la conséquence de la formation d'un duplex entre les deux ARN complémentaires (sens et antisens) empêchant ainsi la production de la protéine. Le mécanisme cependant reste obscur. Le complexe ARN-ARN peut interférer soit avec une transcription ultérieure, soit avec la maturation, le transport ou la traduction ou bien encore conduire à une dégradation de l'ARNm.

Une combinaison de ces effets est également possible.

L'invention concerne également tout ARNm codé par une séquence d'ADN selon l'invention, et plus particulièrement:
- l'ARNm codé par la séquence d'ADN représentée par SEQ ID NO 1, ou codé par un fragment ou une séquence dérivée tels que définis ci-dessus, ledit ARNm étant susceptible de coder à son tour pour la CCR présente chez l'eucalyptus, telle que représentée par SEQ ID NO 2, ou pour un fragment de cette CCR ou une protéine dérivée tels que définis ci-dessus, notamment pour la protéine représentée par SEQ ID NO 10,
- l'ARNm codé par la séquence d'ADN représentée par SEQ ID NO 3, ou codé par un fragment ou une séquence dérivée tels que définis ci-dessus, ledit ARNm étant susceptible de coder à son tour pour la CCR présente chez le peuplier, telle que représentée par SEQ ID NO 4, ou pour un fragment de cette CCR ou une protéine dérivée tels que définis ci-dessus,
- l'ARNm codé par la séquence d'ADN représentée par SEQ ID NO 5, ou codé par un fragment ou une séquence dérivée tels que définis ci-dessus, ledit ARNm étant susceptible de coder à son tour pour la CCR présente chez la fétuque, telle que représentée par SEQ ID NO 6, ou pour un fragment de cette CCR ou une protéine dérivée tels que définis ci-dessus,
- l'ARNm codé par la séquence d'ADN représentée par SEQ ID NO 7, ou codé par un fragment ou une séquence dérivée tels que définis ci-dessus, ledit ARNm étant susceptible de coder à son tour pour la CCR présente chez le tabac, telle que représentée par SEQ ID NO 8, ou pour un fragment de cette CCR ou une protéine dérivée tels que définis ci-dessus.

L'invention a également pour objet tout ARNm antisens, tel que défini ci-dessus, caractérisé en ce qu'il comprend des nucléotides complémentaires de la totalité ou d'une partie seulement des nucléotides constituant un ARNm tel que décrit ci-dessus selon l'invention, ledit ARNm antisens étant susceptible de s'hybrider (ou de s'apparier) avec ce dernier.

A ce titre, l'invention vise plus particulièrement les ARNm antisens codés par des séquences d'ADN selon l'invention, comprenant au moins une région de 50 bases homologues à celles d'une région des séquences complémentaires des séquences d'ADN susmentionnées de l'invention.

Il n'y a pas de limite supérieure de taille pour les séquences d'ADN codant pour un ARN antisens selon l'invention; elles peuvent être aussi longues que celles du messager normalement produit dans les cellules, voire aussi longues que la séquence d'ADN génomique codant pour l'ARNm de la CCR.

Avantageusement, de telles séquences d'ADN codant pour un ARN antisens selon l'invention, comprennent entre environ 100 et environ 1000 paires de bases.

L'invention a plus particulièrement pour objet toute séquence antisens comprenant un (ou plusieurs) ARNm antisens tel(s) que décrit(s) ci-dessus, ou fragment(s) de cet (ces) ARNm antisens, et une (ou plusieurs) séquence(s) correspondant à un (ou plusieurs) domaine(s) catalytique(s) d'un ribozyme.

A ce titre, l'invention vise plus particulièrement toute séquence antisens telle que décrite ci-dessus, comprenant le domaine catalytique d'un ribozyme flanqué de part et d'autre par des bras d'environ 8 bases complémentaires des séquences bordant un motif GUX (X représentant C, U ou A) comprises dans l'un des ARNm de l'invention décrits ci-dessus (encore désignés ARN cibles) (Haseloff J., et Gerlach W. L., 1988, Nature, **334:** 585-591).

L'invention concerne également toute séquence d'ADN susceptible de coder pour une séquence antisens telle que décrite ci-dessus comprenant au moins un domaine catalytique d'un ribozyme lié à un ou plusieurs ARNm antisens de l'invention, ou fragment(s) d'ARNm antisens (avantageusement des fragments d'environ 8 bases tels que décrits ci-dessus).

L'invention a plus particulièrement pour objet encore toute séquence antisens comprenant

L'invention a plus particulièrement pour objet:
- tout ARNm antisens, tel que décrit ci-dessus, caractérisé en ce qu'il est codé par la séquence nucléotidique complémentaire de celle représentée par SEQ ID NO 1, ledit ARNm antisens étant susceptible de s'hybrider avec l'ARNm codé par la séquence d'ADN représentée par SEQ ID NO 1,
- tout ARNm antisens, tel que décrit ci-dessus, caractérisé en ce qu'il est codé par la séquence nucléotidique complémentaire de celle représentée par SEQ ID NO 3, ledit ARNm antisens étant susceptible de s'hybrider avec l'ARNm codé par la séquence d'ADN représentée par SEQ ID NO 3,
- tout ARNm antisens, tel que décrit ci-dessus, caractérisé en ce qu'il est codé par la séquence nucléotidique complémentaire de celle représentée par SEQ ID NO 5, ledit ARNm antisens étant susceptible de s'hybrider avec l'ARNm codé par la séquence d'ADN représentée par SEQ ID NO 5,
- tout ARNm antisens, tel que décrit ci-dessus, caractérisé en ce qu'il est codé par la séquence nucléotidique complémentaire de celle représentée par SEQ ID NO 7, ledit ARNm antisens étant susceptible de s'hybrider avec l'ARNm codé par la séquence d'ADN représentée par SEQ ID NO 7,
- tout ARNm antisens, tel que décrit ci-dessus, caractérisé en ce qu'il est codé par la séquence nucléotidique complémentaire de celle représentée par SEQ ID NO 9, ledit ARNm antisens étant susceptible de s'hybrider avec l'ARNm codé par la séquence d'ADN représentée par SEQ ID NO 9.

L'invention concerne également les polypeptides recombinants codés par les séquences d'ADN de l'invention, lesdits polypeptides recombinants présentant une activité enzymatique équivalente à celle des CCR chez les plantes, et plus particulièrement les CCR recombinantes codées par les séquences représentées par SEQ ID NO 1, SEQ ID NO 3, SEQ ID NO 5 et SEQ ID NO 7, ou par des séquences dérivées de ces dernières selon l'invention, notamment par SEQ ID NO 9.

L'invention a plus particulièrement pour objet les polypeptides recombinants, et notamment les CCR recombinantes, tels qu'obtenus par transformation de cellules végétales en intégrant de façon stable dans leur génome, une séquence nucléotidique recombinante telle que définie ci-après, contenant une séquence d'ADN selon l'invention, notamment à l'aide d'un vecteur tel que décrit ci-après.

Par l'expression "polypeptides recombinants", on doit entendre toute molécule possédant une chaîne polypeptidique susceptible d'être produite par génie génétique, par l'intermédiaire d'une phase de transcription de l'ADN du gène correspondant, ce qui conduit à l'obtention d'ARN qui est par la suite transformé en ARNm (par suppression des introns), ce dernier étant ensuite traduit par les ribosomes, sous forme de protéines, le tout étant effectué sous contrôle d'éléments de régulation appropriés à l'intérieur d'une cellule hôte. Par conséquent, l'expression "polypeptides recombinants" utilisée n'exclut pas la possibilité que lesdits polypeptides comprennent d'autres groupements, tels que les groupements glycosylés.

Bien entendu, le terme "recombinant" indique que le polypeptide a été produit par génie génétique, car il résulte de l'expression, dans un hôte cellulaire approprié, de la séquence nucléotidique correspondante qui a été auparavant introduite dans un vecteur d'expression utilisé pour transformer ledit hôte cellulaire. Toutefois, ce terme "recombinant" n'exclut pas la possibilité que le polypeptide soit produit par un procédé différent, par exemple par synthèse chimique classique selon les méthodes connues utilisées pour la synthèse de protéines, ou par clivage protéolytique de molécules de plus grande taille.

L'invention concerne plus particulièrement la CCR telle que présente dans les cellules d'eucalyptus et représentée par SEQ ID NO 2, la CCR telle que présente dans les cellules de peuplier et représentée par SEQ ID NO 4, la CCR telle que présente dans les cellules de fétuque élevée et représentée par SEQ ID NO 6, ou la CCR telle que présente dans les cellules de tabac et représentée par SEQ ID NO 8, ou toute protéine dérivée de ces dernières, notamment par addition, et/ou suppression, et/ou substitution d'un ou plusieurs acides aminés, notamment la protéine dérivée de la CCR d'eucalyptus et représentée par SEQ ID NO 10, ou tout fragment issu desdites CCR ou de leurs séquences dérivées, lesdits fragments et séquences dérivées étant susceptibles de posséder une activité enzymatique équivalente à celle des CCR susmentionnées.

L'invention a également pour objet les séquences nucléotidiques codant pour la CCR représentée par SEQ ID NO 2, SEQ ID NO 4, SEQ ID NO 6, SEQ ID NO 8, ou SEQ ID NO 10, ou toute séquence dérivée ou fragment de ces dernières, tels que définis ci-dessus, lesdites séquences nucléotidiques étant caractérisées en ce qu'elles correspondent à tout ou partie des séquences représentées par SEQ ID NO 1, SEQ ID NO 3, SEQ ID NO 5, SEQ ID NO 7, ou SEQ ID NO 9 respectivement, ou à toute séquence dérivée de ces dernières par dégénérescence du code génétique, et étant néanmoins susceptibles de coder pour les CCR ou séquence dérivée ou fragment de ces dernières, tels que définis ci-dessus.

L'invention vise également les complexes formés entre les ARNm antisens, tels que décrits ci-dessus, et les ARNm selon l'invention, susceptibles de coder pour tout ou partie d'une CCR chez les plantes.

L'invention a plus particulièrement pour objet le complexe formé entre l'ARNm codé par la séquence SEQ ID NO 1 et l'ARNm antisens codé par la séquence complémentaire de la séquence SEQ ID NO 1, l'ARNm codé par la séquence SEQ ID NO 3 et l'ARNm antisens codé par la séquence complémentaire de la séquence SEQ ID NO 3, l'ARNm codé par la séquence SEQ ID NO 5 et l'ARNm antisens codé par la séquence complémentaire de la séquence SEQ ID NO 5, l'ARNm codé par la séquence SEQ ID NO 7 et l'ARNm antisens codé par la séquence complémentaire de la séquence SEQ ID NO 7, l'ARNm codé par la séquence SEQ ID NO 9 et l'ARNm antisens codé par la séquence complémentaire de SEQ ID NO 9.

L'invention a également pour objet toute séquence nucléotidique recombinante contenant une (ou plusieurs) région(s) codante(s), cette (ces) région(s) codante(s) étant constituée(s):
- d'une séquence nucléotidique codant pour un ARNm, cet ARNm codant lui-même pour une CCR chez les plantes, ou d'un fragment de la séquence nucléotidique susmentionnée, ce fragment codant pour un ARNm, cet ARNm codant lui-même pour un fragment d'une CCR chez les plantes, ce fragment de CCR présentant une activité enzymatique équivalente à celle de la CCR susmentionnée, ou d'une séquence nucléotidique dérivée de la séquence nucléotidique susmentionnée, ou dérivée du fragment susmentionné, notamment par mutation et/ou addition, et/ou suppression, et/ou substitution d'un ou plusieurs nucléotides, cette séquence dérivée codant pour un ARNm, cet ARNm codant lui-même pour une protéine dérivée présentant une activité enzymatique équivalente à celle de la CCR susmentionnée, ou
- d'une séquence nucléotidique complémentaire de tout ou partie de la séquence nucléotidique codant pour un ARNm, ou du fragment de cette séquence, ou de la séquence dérivée de ces derniers, tels que définis ci-dessus, cette séquence complémentaire codant pour un ARNm antisens susceptible de s'hybrider avec l'un des ARNm susmentionnés.

L'invention a plus particulièrement pour objet toute séquence nucléotidique recombinante (ou ADN recombinant), caractérisée en ce qu'elle comprend au moins une séquence d'ADN selon l'invention, choisie parmi celles décrites ci-dessus, ladite séquence d'ADN étant insérée dans une séquence hétérologue.

L'invention concerne plus particulièrement toute séquence nucléotidique recombinante telle que décrite ci-dessus, comprenant, à titre de région codante, la séquence nucléotidique représentée par SEQ ID NO 1, ou par SEQ ID NO 3, ou par SEQ ID NO 5, ou par SEQ ID NO 7, ou tout fragment ou une séquence nucléotidique dérivée de ces dernières, tels que définis ci-dessus, lesdites séquences nucléotidiques ou ledit fragment étant insérés dans une séquence hétérologue, et étant susceptibles de coder pour la CCR représentée par SEQ ID NO 2, ou par SEQ ID NO 4, ou par SEQ ID NO 6, ou par SEQ ID NO 8 respectivement, ou pour un fragment de ces CCR, ou pour une protéine dérivée de ces dernières, tels que définis ci-dessus.

L'invention concerne plus particulièrement encore, toute séquence nucléotidique recombinante comprenant, à titre de région codante, une séquence nucléotidique complémentaire de celle représentée par SEQ ID NO 1, ou par SEQ ID NO 3, ou par SEQ ID NO 5, ou par SEQ ID NO 7, ou tout fragment ou toute séquence nucléotidique dérivée de cette séquence complémentaire, tels que définis ci-dessus, lesdites séquences complémentaires ou ledit fragment étant insérés dans une séquence hétérologue, et étant susceptibles de coder pour un ARNm antisens capable de s'hybrider avec tout ou partie de l'ARNm codant pour une CCR chez les plantes, et plus particulièrement avec tout ou partie de l'ARNm codant pour la CCR représentée par SEQ ID NO 2, ou par SEQ ID NO 4, ou par SEQ ID NO 6, ou par SEQ ID NO 8 respectivement.

Les ADN recombinants selon l'invention sont davantage caractérisés en ce qu'ils comprennent les éléments nécessaires pour réguler l'expression de la séquence nucléotidique codant pour une CCR, ou de sa séquence complémentaire codant pour un ARNm antisens selon l'invention, notamment un promoteur et un terminateur de la transcription de ces séquences.

Parmi les différents promoteurs susceptibles d'être utilisés dans les constructions d'ADN recombinants selon l'invention, on peut citer:
- le promoteur endogène contrôlant l'expression de la CCR chez une plante, notamment le promoteur situé en amont de la séquence représentée par SEQ ID NO 1 chez l'eucalyptus, ou
- des promoteurs de type constitutif à forte expression, exemples: ³⁵S CAMV (décrit dans Benfey et al. (1990), EMBO J., **9** (6), 1677-1684), EF1α (promoteur du gène d'un facteur d'élongation dans la synthèse protéique décrit par Curie et al. (1991), Nucl. Acids Res., **19**, 1305-1310),
- des promoteurs de type spécifique à expression particulière dans des tissus individuels, exemples: promoteur CAD (décrit par Feuillet C. (1993), Thèse de l'Université de Toulouse III), promoteur GRP 1-8 (décrit par Keller et Baumgartner, (1991), Plant Cell., 3, 1051-1061) à expression dans des tissus vasculaires spécifiques.

L'invention concerne également toute séquence nucléotidique recombinante telle que décrite ci-dessus, et comprenant également à titre de région codante au moins une séquence nucléotidique codant pour tout ou partie d'un ARNm codant lui-même pour une autre enzyme que la CCR, qui se trouve être impliquée dans une étape de la biosynthèse des lignines chez les plantes, notamment l'ARNm codant pour l'alcool cinnamylique deshydrogénase (CAD), ou comprenant également à titre de région codante au moins une séquence nucléotidique codant pour tout ou partie d'un ARNm antisens susceptible de s'hybrider avec l'ARNm susmentionné, notamment avec l'ARNm codant pour la CAD.

Les séquences nucléotidiques recombinantes susmentionnées de l'invention sont avantageusement obtenues à partir de vecteurs dans lesquels sont insérées les séquences d'ADN codant pour une enzyme nécessaire à la biosynthèse des lignines chez les plantes.

A ce titre, l'invention a plus particulièrement pour objet le vecteur dénomme pEUCCR, comprenant la séquence représentée par SEQ ID NO 1 clonée dans le vecteur Bluescript, et déposé en culture dans des cellules de *E*. *coli* DH5α à la Collection Nationale de Culture de Micro-organismes (CNCM) de l'Institut Pasteur à Paris (France), le 17 mars 1994 sous le n° I-1405.

Les vecteurs susmentionnés sont digérés à l'aide d'enzymes de restriction appropriées afin de récupérer lesdites séquences d'ADN qui s'y trouvent insérées.

Ces dernières sont ensuite insérées en aval d'un promoteur approprié, et en amont d'un terminateur approprié de l'expression, au sein des ADN recombinants selon l'invention.

L'invention vise plus particulièrement les ADN recombinants comprenant la séquence représentée par SEQ ID NO 1, codant pour l'ARNm codant lui-même pour la CCR représentée par SEQ ID NO 2, tels qu'obtenus par digestion du vecteur pEUCCR susmentionné, récupération de la séquence d'ADN de l'invention et insertion de cette dernière dans le sens 5' → 3', au sein d'une séquence d'ADN hétérologue comprenant un promoteur et un terminateur de l'expression de ladite séquence.

L'invention a également plus particulièrement pour objet les ADN recombinants comprenant la séquence complémentaire de la séquence SEQ ID NO 1, codant pour un ARNm antisens capable de s'hybrider avec l'ARNm codant pour la CCR représentée par SEQ ID NO 2, tels qu'obtenus par digestion du vecteur pEUCCR susmentionné, récupération de la séquence d'ADN de l'invention, et insertion de cette dernière en sens inverse, c'est-à-dire dans le sens 3' → 5', au sein d'une séquence d'ADN hétérologue comprenant un promoteur et un terminateur de l'expression de la séquence complémentaire.

A titre d'exemple de terminateur utilisable dans de telles constructions, on peut citer l'extrémité 3' du gène de la nopaline synthase d'*Agrobacterium tumefaciens*.

Ainsi, d'une manière générale, les séquences nucléotidiques recombinantes selon l'invention, contenant une séquence d'ADN codant pour une CCR (ou fragment de CCR ou protéine dérivée), et/ou d'autres enzymes nécessaires à la biosynthèse des lignines, sont obtenues par récupération de ladite séquence d'ADN à partir des vecteurs susmentionnés, et insertion de cette séquence dans la séquence hétérologue, tandis que les séquences nucléotidiques recombinantes contenant une séquence d'ADN codant pour un ARNm antisens selon l'invention, sont obtenus par récupération de la séquence d'ADN susmentionnée et insertion en sens inverse de cette dernière dans ladite séquence hétérologue.

A titre d'illustration, on peut utiliser tout ou partie de l'ADN complémentaire (ADNc) représenté par SEQ ID NO 1, SEQ ID NO 3, SEQ ID NO 5, SEQ ID NO 7 ou SEQ ID NO 9, pour la construction des ADN recombinants susmentionnés, ou bien tout ou partie du clone génomique correspondant à une CCR (qui correspond aux ADNE susmentionnés + d'éventuels introns). Ce clone génomique peut être obtenu en utilisant les ADNc comme sondes pour cribler une banque génomique, cette dernière étant elle-même obtenue suivant la méthode décrite par Sambrook, Fritsch et Maniatis, Molecular Cloning Laboratory Manual, Cold Spring Harbour Laboratory Press, 1989.

L'invention a également pour objet tout vecteur recombinant, utilisable pour la transformation de plantes, caractérisé en ce qu'il comprend une séquence nucléotidique recombinante choisie parmi celles décrites ci-dessus, selon l'invention, intégrée dans l'un des sites de son génome non essentiels pour sa réplication.

Parmi les vecteurs recombinants susmentionnés utilisables pour la transformation de plantes, on peut citer: les vecteurs binaires dérivés de pBIN 19 (Bevan et al., (1984), Nucl. Acids Res., **12** (22), 8711-8721).

Des exemples de construction de vecteurs recombinants selon l'invention sont décrits dans la description détaillée qui suit de l'invention.

La présente invention a également pour objet un procédé de régulation de la biosynthèse des lignines chez les plantes, soit par diminution, soit par augmentation des quantités de lignines produites, par rapport aux quantités normales de lignines produites chez ces plantes, ledit procédé comprenant une étape de transformation de cellules de ces plantes à l'aide d'un vecteur contenant:
- une séquence nucléotidique codant pour un ARNm, cet ARNm codant lui-même pour une CCR chez les plantes, ou d'un fragment de la séquence nucléotidique susmentionnée, ce fragment codant pour un ARNm, cet ARNm codant lui-même pour un fragment d'une CCR chez les plantes, ce fragment de CCR présentant une activité enzymatique équivalente à celle de la CCR susmentionnée, ou d'une séquence nucléotidique dérivée de la séquence nucléotidique susmentionnée, ou dérivée du fragment susmentionné, notamment par mutation et/ou addition, et/ou suppression, et/ou substitution d'un ou plusieurs nucléotides, cette séquence dérivée codant pour un ARNm, cet ARNm codant lui-même pour une protéine dérivée présentant une activité enzymatique équivalente à celle de la CCR susmentionnée, ou
- une séquence nucléotidique complémentaire de tout ou partie de la séquence nucléotidique codant pour un ARNm, ou du fragment de cette séquence, ou de la séquence dérivée de ces derniers, tels que définis ci-dessus, cette séquence complémentaire codant pour un ARNm antisens susceptible de s'hybrider avec l'un des ARNm susmentionnés,
ladite transformation étant effectuée notamment à l'aide d'un vecteur tel que décrit ci-dessus.

L' invention a plus particulièrement pour objet un procédé de diminution de la quantité de lignines produites par biosynthèse chez les plantes, ce procédé étant effectué par transformation du génome de ces plantes, en y incorporant:
- au moins une séquence d'ADN selon l'invention telle que décrite ci-dessus, codant pour un ARNm antisens susceptible de s'hybrider avec tout ou partie de l'ARNm codant pour la CCR représentée par SEQ ID NO 2, SEQ ID NO 4, SEQ ID NO 6 ou SEQ ID NO 8, ou pour une protéine dérivée de ces dernières telle que définie ci-dessus, notamment pour la protéine représentée par SEQ ID NO 10,
- et, le cas échéant, au moins une séquence d'ADN codant pour un ARNm antisens capable de s'hybrider à un ARNm codant pour une autre enzyme que la CCR, qui se trouve être impliquée dans une étape de la biosynthèse des lignines chez les plantes, notamment l'ARNm codant pour la CAD,
ladite transformation étant réalisée:
- soit à l'aide d'un vecteur recombinant tel que décrit ci-dessus, contenant une séquence d'ADN codant pour un ARNm antisens capable de s'hybrider avec l'ARNm codant pour la CCR ou pour une protéine dérivée, telle que définie ci-dessus, et, le cas échéant, contenant une ou plusieurs séquence(s) d'ADN codant pour un ARNm antisens capable de s'hybrider à un ARNm codant pour une autre enzyme que la CCR telle que définie ci-dessus,
- soit à l'aide de plusieurs vecteurs recombinants dont l'un au moins contient une séquence d'ADN codant pour un ARNm antisens capable de s'hybrider avec l'ARNm codant pour la CCR ou pour une protéine dérivée, telle que définie ci-dessus, tandis que l' (ou les) autre(s) vecteur(s) recombinant(s) contien(nen)t une séquence d'ADN codant pour un ARNm antisens capable de s'hybrider à un ARNm codant pour une autre enzyme que la CCR, telle que définie ci-dessus.

Un autre procédé de diminution de la quantité de lignines produites par biosynthèse chez les plantes, est celui réalisé par transformation du génome de ces plantes, en y incorporant:
- au moins une séquence d'ADN selon l'invention représentée par SEQ ID NO 1, SEQ ID NO 3, SEQ ID NO 5 ou SEQ ID NO 7, ou un fragment ou une séquence dérivée de cette dernière, tels que définis ci-dessus, notamment la séquence représentée par SEQ ID NO 9,
- et, le cas échéant, au moins une séquence d'ADN codant pour tout ou partie d'une autre enzyme que la CCR, qui se trouve être impliquée dans une étape de la biosynthèse des lignines chez les plantes, notamment une séquence d'ADN codant pour tout ou partie de la CAD,
ladite transformation étant réalisée:
- soit à l'aide d'un vecteur recombinant tel que décrit ci-dessus, contenant la séquence d'ADN selon l'invention susmentionnée, ou un fragment ou une séquence dérivée de cette dernière, tels que définis ci-dessus, et, le cas échéant, contenant une ou plusieurs séquence(s) d'ADN codant pour tout ou partie d'une enzyme autre que la CCR, telle que définie ci-dessus,
- soit à l'aide de plusieurs vecteurs recombinants dont l'un au moins contient une séquence d'ADN selon l'invention susmentionnée, ou un fragment ou une séquence dérivée de cette dernière, tels que définis ci-dessus, tandis que l' (ou les) autre(s) vecteur(s) recombinant(s) contien(nen)t une séquence d'ADN codant pour tout ou partie d'une enzyme autre que la CCR, telle que définie ci-dessus.

Cette dernière méthode fait appel au mécanisme de co-suppression. La co-suppression a été observée quand des copies du gène endogène ont été introduites dans le génome. Bien que le mécanisme de la co-suppression soit actuellement inconnu, une des hypothèses les plus fréquemment retenue est que la régulation négative de l'expression du gène viendrait de la production d'une faible proportion d'ARN antisens dérivée d'un transgène à travers une lecture du "mauvais" brin du transgène (Grierson et al., Trends Biotech., **9**: 122-123).

L'invention a également pour objet un procédé de diminution de la quantité de lignines produites par biosynthèse chez les plantes, ce procédé étant réalisé par transformation du génome de ces plantes en y incorporant une séquence d'ADN telle que décrite ci-dessus selon l'invention, codant pour une séquence antisens comprenant un (ou plusieurs) domaine(s) catalytique(s) d'un ribozyme lié(s) à un (ou plusieurs) ARNm antisens, ou fragment(s) d'ARNm antisens de l'invention, ladite transformation étant réalisée à l'aide d'un vecteur recombinant comprenant une séquence nucléotidique recombinante selon l'invention contenant elle-même la séquence d'ADN susmentionnée.

Il est important de noter que les méthodes susmentionnées permettent d'aboutir à des plantes transformées présentant des niveaux différents de réduction de l'activité CCR (selon le niveau d'insertion de la séquence d'ADN codant pour l'ARNm antisens, le nombre de copies de cette séquence d'ADN intégrée dans le génome...), et donc des teneurs en lignines.

Le choix des transformants permettra donc une modulation contrôlée des teneurs en lignines compatible avec un développement normal de la plante.

D'une manière générale, si l'on considère que la teneur moyenne normale en lignines d'une plante varie entre environ 15% et environ 35% en poids de matière sèche, la réduction de la teneur en lignines résultant de la mise en oeuvre d'un des procédés susmentionnés, est avantageusement telle que les plantes ainsi transformées présentent une teneur moyenne en lignines variant entre environ 10% et environ 30%, ou encore entre environ 12% et environ 32%.

A titre d'illustration, la teneur en lignines d'une plante peut être mesurée selon une variante de la méthode de Johnson et al., (1961), T.A.P.P.I., **44**, 793-798, qui est décrite en détail dans Alibert et Baudet (1979), Physiol., Veg., **17** (1), 67-74, et dont les principales étapes sont les suivantes: après obtention d'une poudre alcool benzène contenant les lignines du matériel végétal, les lignines sont solubilisées par le bromure d'acétyle et dosées en fonction de leur absorption dans l'ultraviolet.

L'invention vise plus particulièrement l'application des procédés susmentionnés de diminution des teneurs en lignines dans les plantes, à l'obtention de plantes fourragères transformées génétiquement, présentant des teneurs en lignines réduites par rapport aux teneurs normales en lignines chez ces plantes, et dont la digestibilité se trouve être ainsi améliorée par rapport à ces mêmes plantes non transformées.

Parmi les principales plantes fourragères susceptibles d'être transformées dans le cadre de la présente invention, on peut citer: la luzerne, la fétuque, le maïs destiné à l'ensilage, etc...

L'invention concerne également l'application des procédés susmentionnés de diminution des teneurs en lignines chez les plantes, à l'obtention de plantes, et plus particulièrement d'arbres, transformés génétiquement, présentant des teneurs en lignines réduites par rapport aux teneurs normales en lignines chez ces plantes, ces plantes ou arbres étant particulièrement avantageux à utiliser dans le cadre de la production de la pâte à papier.

Un troisième domaine potentiel d'application des procédés susmentionnés de régulation négative de l'expression du gène de la CCR, concerne la stimulation de la croissance des plantes transformées. Divers arguments soulignent (Sauter et Kende, 1992, Plant and Cell Physiology, **33** (8):1089), qu'une lignification précoce et rapide est une frein au grandissement cellulaire et donc à la croissance des végétaux. Ainsi la mise en oeuvre des procédés susmentionnés est susceptible de permettre pour les plantes ainsi transformées à lignification réduite une meilleure croissance et donc de meilleurs rendements.

L'invention concerne également un procédé d'augmentation de la quantité de lignines produites par biosynthèse chez les plantes, ce procédé étant effectué par transformation du génome de ces plantes, en y incorporant:
- au moins une séquence d'ADN selon l'invention représentée par SEQ ID NO 1, SEQ ID NO 3, SEQ ID NO 5 ou SEQ ID NO 7, ou un fragment ou une séquence dérivée de cette dernière, tels que définis ci-dessus, notamment la séquence représentée par SEQ ID NO 9,
- et, le cas échéant, au moins une séquence d'ADN codant pour tout ou partie d'une autre enzyme que la CCR, qui se trouve être impliquée dans une étape de la biosynthèse des lignines chez les plantes, notamment une séquence d'ADN codant pour tout ou partie de la CAD,
ladite transformation étant réalisée:
- soit à l'aide d'un vecteur recombinant tel que décrit ci-dessus, contenant la séquence d'ADN selon l'invention susmentionnée, ou un fragment ou une séquence dérivée de cette dernière, tels que définis ci-dessus, et, le cas échéant, contenant une ou plusieurs séquences) d'ADN codant pour tout ou partie d'une enzyme autre que la CCR, telle que définie ci-dessus,
- soit à l'aide de plusieurs vecteurs recombinants dont l'un au moins contient une séquence d'ADN selon l'invention susmentionnée, ou un fragment ou une séquence dérivée de cette dernière, tels que définis ci-dessus, tandis que l' (ou les) autre(s) vecteur(s) recombinant(s) contien(nen)t une séquence d'ADN codant pour tout ou partie d'une enzyme autre que la CCR, telle que définie ci-dessus.

D'une manière générale, toujours si l'on considère que la teneur moyenne normale en lignines d'une plante varie entre environ 15% et environ 35% en poids de matière sèche, l'augmentation de la teneur en lignines résultant de la mise en oeuvre du procédé susmentionné, est avantageusement telle que les plantes ainsi transformées présentent une teneur moyenne en lignines variant entre environ 20% et environ 40%, entre environ 18% et environ 38%.

L'invention vise plus particulièrement l'application du procédé susmentionné d'augmentation des teneurs en lignines dans les plantes (encore désigné procédé de surexpression du gène de la CCR), à l'obtention de plantes transformées génétiquement, présentant des teneurs en lignines augmentées par rapport aux teneurs normales en lignines chez ces plantes, et dont les propriétés de résistance à des attaques de l'environnement, notamment à des attaques parasitaires, se trouvent être ainsi améliorées par rapport à ces mêmes plantes non transformées. Il est particulièrement avantageux dans ce dernier cas, d'utiliser en association avec le gène CCR, ou une séquence dérivée, dans les vecteurs susmentionnés, des promoteurs spécifiques particulièrement exprimés dans les tissus de surface et/ou en réponse à la blessure.

Par ailleurs, l'invention concerne également l'application du procédé susmentionné de surexpression du gène de la CCR, à l'amélioration de la croissance des plantes ainsi transformées génétiquement, notamment dans certains domaines tels que l'horticulture ou l'arboriculture, où il est souhaitable d'obtenir des plantes de dimension réduite.

Enfin, les cycles benzéniques de la lignine ont une plus grande énergie intrinsèque que les chaînes aliphatiques des résidus glucose de la cellulose. Ainsi, l'augmentation de la proportion de lignines chez les végétaux utilisés comme combustibles, selon le procédé susmentionné de l'invention, permet d'améliorer le potentiel énergétique de ces végétaux combustibles ainsi transformés.

Dans les deux cas de régulation négative ou de surexpression de la CCR, il est tout à fait envisageable que la modulation de cette activité se répercute sur la teneur en lignines des plantes transformées. En effet, la CCR dont le niveau d'activité est très bas dans la plante, semble constituer l'enzyme régulatrice de la synthèse des lignines.

S'agissant des techniques de transformation utilisées pour la mise en oeuvre d'un des procédés décrits ci-dessus de l'invention, on aura avantageusement recours aux techniques suivantes:
A) La technologie de transformation par l'intermédiaire du plasmide Ti d'*Agrobacterium tumefaciens* décrite par Bevan (1984) Nucleic Acid Research, **12**: 8711-8721. Elle fait appel essentiellement à la méthode de co-culture, et fait intervenir une co-transformation avec un gène de sélection pour pouvoir repérer les transformants.
   Elle est particulièrement applicable aux dicotylédones, ex.: tabac, luzerne, colza.
B) La technique de transfert direct de gènes par biolistique décrite en détail par (Zumbrum et al., 1989, Technique **1**, 204-216; Sanford et al., 1991, Technique **3**, 3-16).

Cette technique implique l'association de l'ADN recombinant selon l'invention à des microparticules d'or ou de tungstène qui sont propulsées à l'aide d'un canon à particules sur le tissu à transformer. Elle sera particulièrement appliquée à la transformation d'espèces réfractaires aux agrobactéries.

Dans les deux cas susmentionnés, la vérification de la présence de l'ADN recombinant selon l'invention sera réalisée par des expériences d'hybridation de type southern et d'amplification génique (polymerase chain réaction), à l'aide se sondes et d'amorces oligonucléotidiques issues notamment de la séquence SEQ ID NO 1, SEQ ID NO 3, SEQ ID NO 5, SEQ ID NO 7 ou SEQ ID NO 9.

L'invention concerne également les cellules de plantes transformées par un vecteur selon l'invention, notamment par les techniques décrites ci-dessus, et comprenant une séquence d'ADN selon l'invention intégrée de façon stable dans leur génome.

L'invention vise également les plantes transformées telles qu'obtenues par culture des cellules transformées susmentionnées.

Les plantes transformées peuvent être ensuite propagées par voie sexuelle ou par voie végétative *in vitro* ou *in natura*.

L'invention a également pour objet les fragments de plantes, notamment fruits, semences, pollen, transformés par incorporation dans leur génome d'une séquence d'ADN selon l'invention, à l'aide des vecteurs recombinants susmentionnés.

L'invention concerne également les anticorps dirigés contre les polypeptides recombinants de l'invention, et plus particulièrement ceux dirigés contre les CCR recombinantes susmentionnées.

De tels anticorps peuvent être obtenus par immunisation d'un animal avec ces polypeptides suivie de la récupération des anticorps formés.

Il va de soi que cette production n'est pas limitée aux anticorps polyclonaux.

Elle s'applique encore à tout anticorps monoclonal produit par tout hybridome susceptible d'être formé, par des méthodes classiques, à partir des cellules spléniques d'un animal, notamment de souris ou de rat, immunisés contre l'un des polypeptides purifiés de l'invention d'une part, et des cellules d'un myélome approprié d'autre part, et d'être sélectionné, par sa capacité à produire des anticorps monoclonaux reconnaissant le polypeptide susmentionné initialement mis en oeuvre pour l'immunisation des animaux.

L'invention vise également l'utilisation des anticorps susmentionnés dirigés contre les polypeptides recombinants de l'invention, pour la mise en oeuvre d'une méthode de détection ou de dosage des CCR chez les plantes, à partir d'échantillons prélevés chez ces dernières.

L'invention sera davantage détaillée dans la description qui suit de l'obtention de la CCR sous forme purifiée chez l'eucalyptus, et de l'ADNc codant pour la CCR d'eucalyptus, de peuplier, de la fétuque élevée et du tabac

### A) Obtention de la CCR d'eucalyptus purifiée, et de l'ADNc codant pour une CCR d'eucalyptus.

### I Purification de la CCR d'eucalyptus.

La CCR a fait l'objet d'un nombre très restreint d'études. Parmi les quelques publications la concernant, on peut citer:
Wengenmayer H., Ebel J., Grisebach H., 1976 - Enzymatic synthesis of lignin precursors, purification and properties of a cinnamoyl CoA: NaDPH reductase from cell suspension cultures from soybean (*Glycine max*), Eur. J. Biochem., **65**: 529-536.
Luderitz T., Grisebach H., 1981 - Enzymatic synthesis of lignin precursors, comparison of cinnamoyl: CoA reductase and cinnamyl alcohol dehydrogenase: NADP dehydrogenase from spruce (*Picea abies* L.) and soybean (*Glycine max L*.), Eur. J. Biochem., **119**: 115-127.
Sarni F., Grand C., Boudet A.M., 1984 - Purification and properties of cinnamoyl: CoA reductase and cinnamyl alcohol dehydrogenase from poplar stems (*Populus x euramericana*). Eur. J. Biochem., **139**: 259-265.

Le travail décrit ci-après a contribué à définir un protocole de purification original, simple et rapide de la CCR d'eucalyptus. Ce protocole est également plus efficace que ceux décrits précédemment dans la littérature.

En effet, il a permis pour la première fois, l'obtention de quantités d'enzyme purifiée à homogénéité, suffisantes pour obtenir des séquences peptidiques internes et conduire à terme au clonage de l'ADNc correspondant.

Toutes les étapes de purification de la CCR ont été réalisées à 4°C.

### 1. Obtention d'un extrait brut de xylème d'eucalyptus.

Le matériel végétal a été obtenu par "grattage" d'une fraction tissulaire enrichie en xylème de branches *d'Eucalyptus gunnii* âgés de 5 ans.

300 g de xylème préalablement congelé dans l'azote liquide ont été réduits en poudre à l'aide d'un moulin à café. Le broyat ainsi obtenu a été homogénéisé dans un litre de tampon d'extraction (100 mM Tris-HCl pH 7.6, 2% PEG 6000, 5 mM DTT, 2% PVPP), filtré sur deux épaisseurs de Miracloth, et amené à 30% de la saturation en sulfate d'ammonium. Après une centrifugation de 30 minutes à 15000xg, le culot obtenu est remis en suspension dans 60 ml de tampon 1 [20 mM Tris-HCl pH 7.5, 5 mM DTT (dithiothreitol), 5% éthylène glycol]. L'extrait ainsi obtenu est "clarifié" par une centrifugation de 15 min à 10 000xg, puis dessalé par passage sur une Sephadex G25 équilibrée dans le tampon 1.

### 2 Chromatographie d'affinité sur Red Sepharose.

L'extrait brut dessalé est déposé sur une colonne d'affinité "Red Sepharose" (1.5 x 19 cm, Pharmacia), équilibrée dans le tampon 1. Après un premier rinçage de la colonne par 50 ml de tampon 1, les protéines sont éluées par un gradient linéaire de Tris de 20 mM à 1.5 M Tris-HCl pH7.5, contenant 5 mM DTT, 5% éthylène glycol. Le volume total du gradient est de 200 ml et le débit de 36 ml/h. les fractions présentant une activité CCR sont regroupées et dessalées par passage sur une colonne de Séphadex G25, équilibrée dans le tampon 1.

### 3. Chromatographie d'échange d'anions sur MonoQ.

Les fractions ainsi regroupées et dessalées sont chromatographiées sur une colonne d'échange d'anions MonoQ (HR 5/5, Pharmacia). L'élution des protéines est effectuée par l'application d'un gradient linéaire de 20 à 300 mM en Tris-HCl pH 7.5, contenant 5% d'éthylène glycol et 5 mM DTT. Le volume total du gradient est de 50 ml et le débit de 1 ml/min. Comme à l'étape précédente, les fractions contenant l'enzyme CCR active sont regroupées et dessalées, mais dans ce cas le tampon d'équilibration des colonnes de Sephadex G25 est un tampon phosphate 20 mM pH 7.6, contenant 5 mM DTT (tampon 2).

### 4. Chromatographie d'affinité sur "Mimetic Red".

Le groupe de fractions CCR ainsi obtenu est déposé sur une colonne Mimetic Red 2 A6XL (ACL, Cambridge). La colonne est préalablement lavée avec 30 ml de tampon 2 contenant 8 mM NAD. Ce lavage a pour but d'éliminer des enzymes fonctionnant spécifiquement avec le NAD comme cofacteur, telle que la malate deshydrogénase qui copurifie avec la CCR dans les étapes précédentes. L'élution spécifique de la CCR est obtenue par application d'un gradient (15 ml) de NADP 0-8 mM dans le tampon 2. Les fractions contenant la CCR pure et active sont conservées à -80°C après addition d'un stabilisateur (éthylène glycol à la concentration finale de 5%).

L'enzyme purifiée ainsi obtenue présente une activité spécifique de 451 nKat/mg de protéine, en utilisant le feruloyl CoA comme substrat. Le rendement obtenu (36 µg de protéine pure pour 300 g de matériel végétal de départ) ne reflète pas la proportion de CCR *in planta*, en effet dans un souci majeur d'éliminer le maximum de contaminants à chaque étape de purification, seules les fractions présentant une très forte activité CCR sont traitées par l'étape suivante. Le facteur de purification obtenu par ce protocole est de 282.

### II Caractérisation de la CCR.

La CCR d'eucalyptus est un monomère de 38kD comme en témoignent les résultats convergents obtenus pour la taille de l'enzyme native par chromatographie d'exclusion sur Superose 6 (Pharmacia), et pour la taille de la sous-unité monomère sur gel d'électrophorèse dénaturant. Le point isoélectrique, estimé par chromatographie sur MonoP (Pharmacia) est proche de 7.

La recherche du pH et du tampon optimum fait ressortir que la mesure de l'activité CCR telle qu'elle a été initialement décrite (Luderitz et Grisebach, 1981), est parfaitement adaptée à la mesure de l'activité CCR d'eucalyptus (tampon phosphate 100 mM, pH 6.25).

La pureté de la CCR, présente à l'état d'une bande unique sur gel d'électrophorèse monodimensionnelle (SDS PAGE) a été confirmée par l'obtention d'une seule tache ("spot") après électrophorèse tridimensionnelle et coloration à l'argent.

### III Obtention de l'ADNc codant pour la CCR d'eucalyptus

Afin de s'affranchir d'un éventuel problème de contamination résiduelle; non détectable, l'enzyme pure a été soumise à une électrophorèse préparative. en conditions semi-dénaturantes et digérée *in situ* dans le gel. La digestion a été réalisée à l'aide d'endolysine C qui coupe spécifiquement les protéines après les résidus lysine, permettant l'obtention de peptides relativement longs. Les peptides résultant de la digestion ont été séparés en phase reverse sur HPLC et certains d'entre eux ont été séquencés à l'aide d'un microséquenceur de protéines (Applied Biosystems 470). Les séquences de ces peptides internes figurent ci-dessous: X représentant tout acide aminé

L'ADNc codant pour la CCR a été obtenu par criblage à l'aide d'oligonucléotides d'une banque d'ADNc construite dans le phage λ ZAPII (vecteur commercialement disponible, Stratagène) à partir de messagers extraits de xylème d'*Eucalyptus gunnii.* 600 000 phages ont été criblés à l'aide d'un groupe d'oligonucléotides dégénérés marqués à l'extrémité 3' au phosphore 32, à l'aide d'une terminal transferase. Les séquences des oligonucléotides utilisés pour le criblage ont été déterminées à partir des séquences peptidiques internes susmentionnées. Ces peptides ayant été générés par coupure à l'endolysine C, une lysine a été rajoutée en première position pour permettre l'élaboration d'oligonucléotides à moindre dégénérescence. En effet, cet acide aminé qui ne peut être codé que par deux codons, fait partie des acides aminés dont le code est le moins dégénéré et par conséquent convient tout à fait à l'élaboration d'oligonucléotides à partir de séquences peptidiques.

Les séquences des oligonucléotides utilisés pour le criblage de la banque de cDNA d'eucalyptus dérivées des acides aminés soulignés (I = inosine), sont indiquées ci-après:

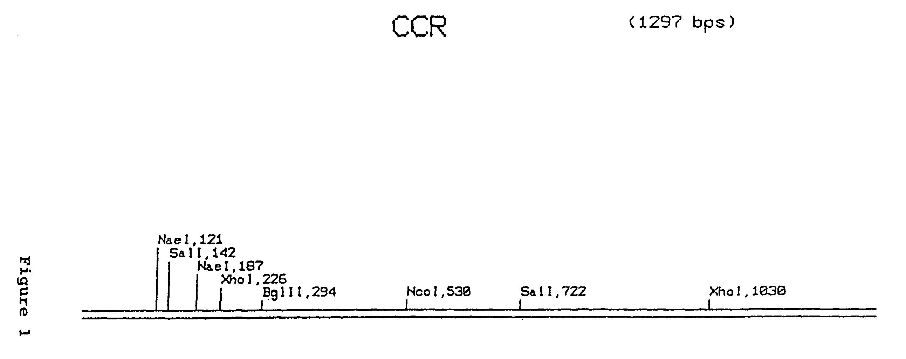

Les conditions d'hybridation utilisées pour le criblage sont les suivantes: la préhybridation est effectuée pendant 6 à 7 heures dans du 5XSSPE, 0.25% poudre de lait écrémé, 0.05% SDS (sodium dodecyl sulfate) à 42°C. L'hybridation est réalisée dans cette même solution, en présence des 4 oligonucléotides marqués en 3' au ddATPα³²P, pendant 24 heures à 42°C. Au terme de ces 24 heures d'hybridation, les filtres sont lavés trois fois pendant 15 minutes dans du 2XSSC, 0.1% SDS puis mis en contact avec un film autoradiographique pendant 24 heures à -80°C. Les phages hybridant avec le groupe d'oligonucléotides ont été purifiés par 2 tours supplémentaires de criblage ("plaque purification"). Une fois purifiés, les six clones positifs ont été testés avec chacun des oligonucléotides pris indépendamment. Un phage a réagi positivement avec les 4 oligonucléotides, il a été traité de manière à "exciser" le plasmide Bluescript recombinant en suivant les indications du fabricant (Stratagène). La carte de restriction de l'insert (codant pour la CCR) contenu dans ce plasmide est schématisée sur la figure 1.

### IV Caractérisation et identification de l'ADNc de la CCR

La séquence en acides aminés (représentée par SEQ ID NO 2) déduite de la séquence nucléotidique (représentée par SEQ ID NO 1) code pour une protéine de 335 acides aminés dont le poids moléculaire est de 36,5 kD et le point isoélectrique d'environ 5,8. Il est important de souligner que toutes les séquences peptidiques obtenues à partir de la CCR purifiée sont retrouvées dans la séquence peptidique déduite de la séquence nucléotidique de l'ADNc.

Des recherches d'homologies avec des clones déjà existants ont été effectuées en utilisant les programmes BLAST et FASTA dans toutes les banques protéiques et nucléiques disponibles. Une homologie significative a été trouvée avec une autre réductase du métabolisme des composés phénoliques, la dihydroflavonol réductase (DFR). L'identité est d'environ 40% et la similarité proche de 20% entre la séquence peptidique déduite de l'ADNc de la CCR et les séquences des diverses dihydroflavonol réductase répertoriées dans les banques, ce qui confirme que le clone identifié est différent d'un clone codant pour une DFR.

### V Production de CCR recombinante active dans E. Coli.

Pour aller plus loin dans l'identification de l'ADNc de la CCR, la protéine recombinante a été produite dans *E. Coli* et son activité enzymatique a été recherchée. Les détails expérimentaux de cette approche sont décrits ci-après.

### 1- Introduction de l'ADNc dans le vecteur d'expression pT7-7.

Afin de pouvoir cloner l'ADNc dans le vecteur d'expression pT7-7 (disponible commercialement), sous le contrôle du promoteur de la T7 polymérase, nous avons du introduire un site Ndel à l'ATG de l'ADNc. Ceci a été réalisé à l'aide d'une Taq polymérase lors d'une réaction d'amplification génique par PCR (Polymerase Chain reaction) entre un oligonucléotide muté et une amorce commerciale, T7, situé sur Bluescript en aval de l'extrémité 3' de l'ADNc. Le produit d'amplification obtenu est digéré par Kpnl, ce site est ensuite réparé à l'aide du fragment klenow de l'ADN polymérase I avant de soumettre le fragment à une digestion par Ndel, puis le fragment obtenu comportant un site Ndel en 5' et une extrémité franche en 3' est insérée à l'aide d'une ADN T4 ligase dans le vecteur pT7-7 préalablement ouvert par Ndel et Smal.

La séquence de l'oligonucléotide muté susmentionné est indiquée ci-après.

Les bases soulignées et en italiques ont été modifiées par rapport à la séquence initiale permettant la création d'un site Ndel (CATATG):

### 2. Surexpression de CCR dans E. Coli BL21

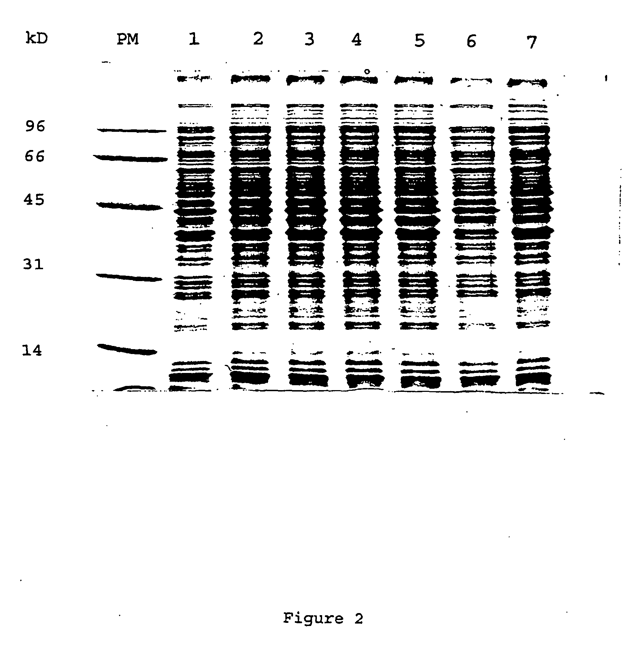

La construction ainsi obtenue est introduite dans la souche *E. Coli*, BL21 (disponible commercialement) qui porte sur son chromosome le gène de la T7 polymérase sous contrôle du promoteur lac UV5, promoteur inductible par l'IPTG. La culture recombinante est cultivée à 37°C jusqu'à obtention d'une DO mesurée de 1 à 600 nm, puis la production de la CCR est induite par l'ajout d'IPTG (0.25% final) dans le milieu de culture. Des prélèvements sont réalisés à différents temps après induction et les cellules sont lysées selon le protocole décrit par Grima-Pettenati et al. (1993). Après centrifugation le surnageant contenant les protéines solubles, est utilisé pour mesurer l'activité CCR et pour visualiser la production de CCR après électrophorèse en conditions dénaturantes. Sur la figure 2, on note l'apparition d'un polypeptide. d'environ 38 kD dont l'intensité croit avec le temps post-induction et qui n'existe pas dans les témoins négatifs (souche BL21 contenant seulement le vecteur pT7-7 sans insert). De plus, la preuve finale de l'identité du clone CCR est apporté par la mesure d'une activité CCR (environ 7 nKat/ml de culture après 3h d'induction à 37°C) dans les extraits protéiques provenant des souches BL21 contenant le pT7-7 + ADNc CCR, seulement.

Légendes des figures:
Figure 1: carte de restriction de l'ADNc codant pour la CCR d' eucalyptus.
Figure 2: révélation au bleu de Coomassie d'un gel d'électrophorèse en conditions dénaturantes (SDS-PAGE) d'extraits protéiques totaux de *E. Coli* BL21 contenant le vecteur pT7-7 sans insert (piste 6 au temps 0 de l'induction par l'IPTG; 7, 3 heures après induction) et le vecteur pT7-7 contenant le cDNA de la CCR (pistes 1, 2, 3, 4, 5, respectivement, temps 0 de l'induction, 30 min, 1, 2, 3 heures après induction), la flèche désigne le monomère CCR.

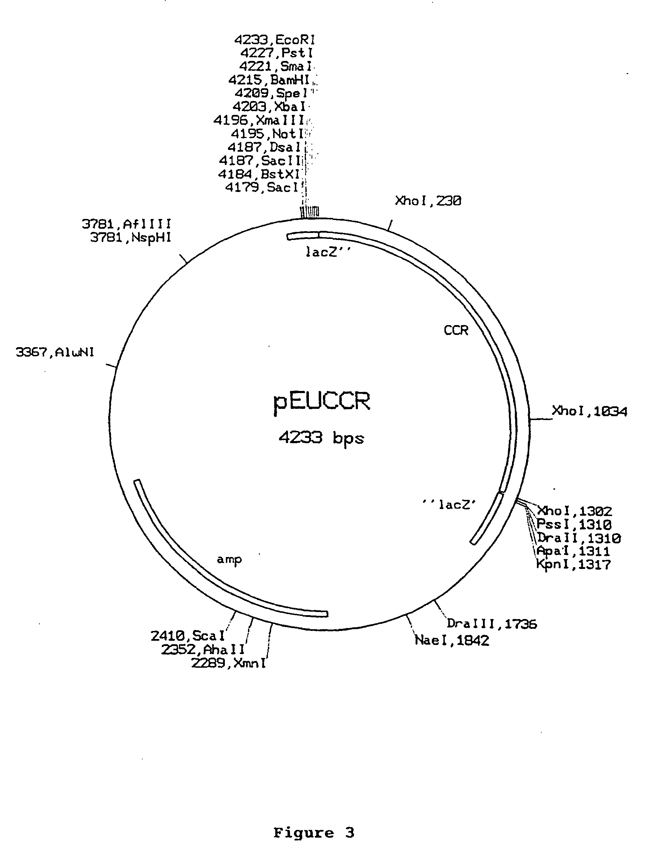

Figure 3: représentation schématique du plasmide pEUCCR contenant la séquence représentée par SEQ ID NO 1 (et identifiée par CCR dans le plasmide pEUCCR).
Figure 4: représentation schématique de la construction d'un vecteur contenant une séquence d'ADN codant pour la CCR d'eucalyptus selon l'invention (ou vecteur CCR sens).
Figure 5: représentation schématique de la construction d'un vecteur contenant une séquence d'ADN codant pour un ARN antisens susceptible de s'hybrider avec l'ARNm codant pour la CCR d'eucalyptus selon l'invention (ou vecteur CCR antisens).

### Concernant l'ADN "source servant à la construction d'un vecteur antisens (ou sens)

L'ARN antisens dérive préférentiellement de la séquence contenue dans le clone pEUCCR. Cette séquence peut être obtenue de différentes manières:
1) en coupant avec des enzymes de restriction appropriées la séquence d'ADN (ADNc) de la CCR contenue dans pEUCCR,
2) en réalisant une amplification génique (PCR) à l'aide d'oligonucléotides définis de manière à synthétiser le fragment d'ADN souhaité.

Le fragment d'ADN ainsi obtenu est clone dans un vecteur d'expression des plantes en aval d'un promoteur et en amont d'un terminateur. Le clonage est réalisé de telle façon que le fragment d'ADN est inséré en orientation inverse par rapport au promoteur. Dans ce nouveau vecteur, le brin qui était initialement le brin matrice devient le brin codant et vice versa.

Le nouveau vecteur code pour un ARN dont la séquence est complémentaire de la séquence de l'ARN messager déduit de la séquence contenue dans pEUCCR.

Ainsi les 2 ARN sont complémentaires de par leur séquence mais aussi de par leur orientation (5'-3').

Comme source d'ADN pour la transcription de l'ARN antisens il est pratique d'utiliser un clone d'ADNc tel que celui contenu dans pEUCCR.

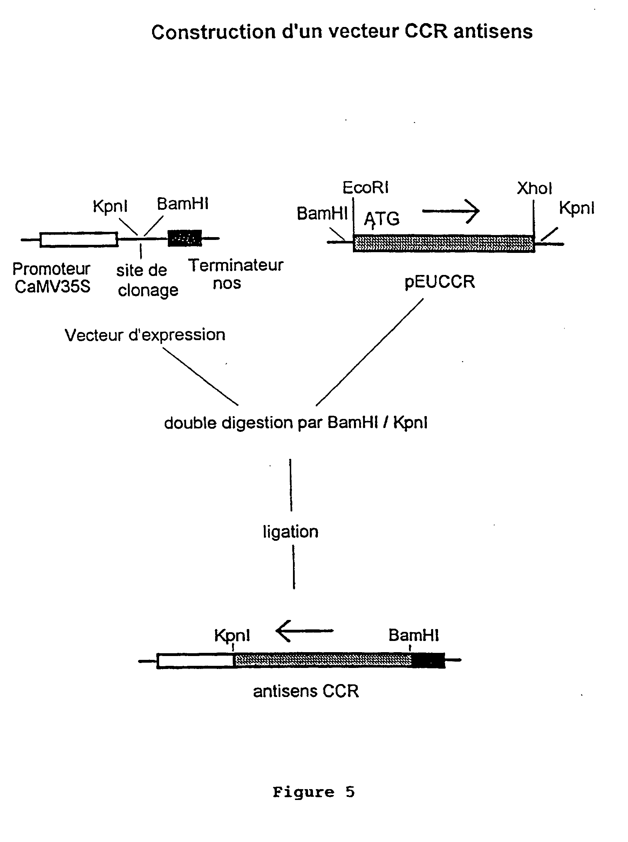

### Exemple de clonage (cf. figure 5)

L'ADNE de la CCR est obtenu par une double digestion (BamHI et KpnI) à partir du vecteur pEUCCR. Le fragment d'ADN ainsi libéré est séparé physiquement du vecteur de clonage par une électrophorèse en gel d'Agarose (Bluescript).

La partie de gel renfermant ce fragment d'ADN est découpée et traitée de façon à obtenir l'ADN purifié (plusieurs méthodes peuvent être utilisées dont la "Low melting Agarose", décrite dans Sambrook et al. susmentionné, le Gene Clean, dont le kit est disponible commercialement).

Le fragment portant les extrémités BamHI et KpnI est "ligué" avec un vecteur d'expression de plantes préalablement digéré par ces mêmes enzymes choisies de façon à ce que le cDNA soit inséré en orientation inverse par rapport au promoteur ³⁵S. Le brin qui sera transcrit dans les plantes sera dans ce cas le brin non codant.

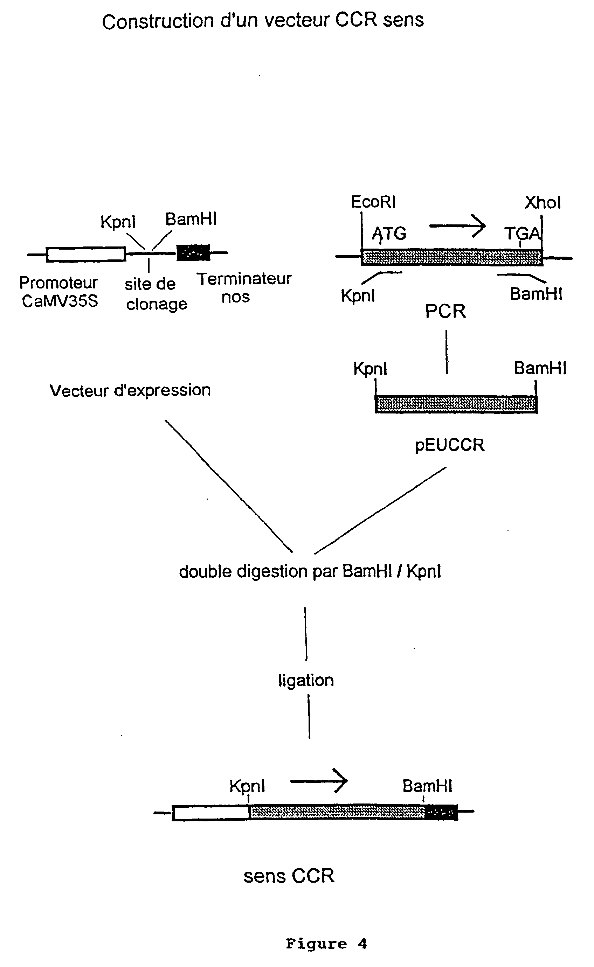

### Exemple de clonage sens (cf. figure 4)

Dans ce cas, il n'existe pas de sites de restriction "pratiques" pour réaliser une fusion traductionnelle avec le promoteur ^{3S}S du vecteur d'expression. De nouveaux sites plus commodes ont été insérés à l'aide de la technique d'amplification génique (PCR). Deux oligonucléotides ont été définis en 5' et 3' de l'ADNc auxquels ont été ajoutées les séquences des sites reconnus par KpnI et BamHI (NB.: ce sont les mêmes sites qui ont été utilisés pour le clonage antisens susmentionné, mais positionnés différemment par rapport à l'orientation 5'-3').

L'amplification génique conduit à l'obtention d'un fragment contenant la totalité de la séquence codante du cDNA flanquée de 2 sites de restriction. La suite de la procédure est identique à celle décrite pour la construction antisens.

Mais, dans ce cas, on a réalisé une fusion du promoteur en phase avec l'ATG de la CCR qui doit conduire à une surexpression de l'ARN messager et donc de la protéine CCR.

### B) Obtention de l'ADNc codant pour la CCR de peuplier.

L'ADNc a été obtenu par criblage d'une banque d'ADNc construite dans le phage lambda ZAPII (Stratagène) à partir de messagers extraits de xylèmes de peupliers âgés de 2 ans (*Populus trichocarpa*). Les phages ont été criblés à l'aide de l'ADNc isolé chez *Eucalyptus gunnii* (fragment Xhol de 804 pb de pEUCCR) marqué par random priming au dCTPα³²p.

Les conditions d'hybridation utilisées pour le criblage sont les suivantes: la préhybridation est effectuée pendant une nuit à 68°C dans 6X SSC, 5 x Denhardt, 0,5% SDS, 100 µg d'ADN de sperme de saumon. L'hybridation est réalisée dans le même tampon sans addition de réactif de Denhardt, pendant une nuit à 60°C. Les membranes sont ensuite lavées 2 fois pendant 30 minutes dans 0,1X SSC, 0,5% SDS à 60°C.

Un clone contenant l'ADNc complet a été purifié, sous-cloné et séquencé dans les deux directions. La séquence d'ADNc est celle représentée par SEQ ID NO 3, et la séquence en acides aminés déduite de cette dernière est celle représentée par SEQ ID NO 4.

La comparaison entre les séquences en acides aminés de la CCR d'eucalyptus est celle du peuplier montre une identité de 83% et une homologie de 93% entre ces deux séquences.

### C) Obtention de l'ADNc codant pour la CCR de fétuque.

L'ADNc a été obtenu par criblage d'une banque d'ADNc construite dans le phage lambda ZAPII (Stratagène) à partir de messagers extraits de feuilles de *Fetusca arundinacea*. 500 000 phages ont été criblés à l'aide de l'ADNc isolé chez *Eucalyptus gunnii* (fragment Xhol de 804 pb de pEUCCR) marqué par random priming au dCTPα³²p.

Les conditions d'hybridation utilisées pour le criblage sont les suivantes: la préhybridation est effectuée pendant 6 heures dans du 5X SSPE, SDS 0,5%, ADN de sperme de saumon 0,1 mg/ml, Ficoll type 400 1 mg/ml, polyvinyipyrrolidone 1 mg/ml, BSA 1 mg/ml à 60°C. L'hybridation est réalisée dans cette même solution, en présence de la sonde marquée au dCTPα³²p, pendant 16 heures à 60°C. Les filtres sont alors lavés 2 fois pendant 15 minutes dans du 2X SPPE, 0,1% SDS à 60°C puis 2 fois dans du 0,5X SSPE, 0,1% SDS à 60°C et enfin mis en contact avec un film autoradiographique pendant 3 jours à -80°C. Les phages ont été purifiés par 2 ou 3 tours supplémentaires de criblage.

Une fois purifiés, 3 clones ont été analysés après que le plasmide pBluescript ait été excisé en suivant les indications du fournisseur (Stratagène).

Le séquençage a permis d'éliminer un clone et de montrer que les 2 autres clones étaient identiques et correspondaient à la séquence nucléotidique représentée par SEQ ID NO 5 codant pour la CCR de fétuque telle que représentée par SEQ ID NO 6.

### D) Obtention de l'ADNc codant pour la CCR de tabac.

L'ADNc a été obtenu par criblage d'une banque d'ADNc construite dans le phage lambda ZAPII (Stratagène) à partir de messagers extraits de tiges de tabac. Les phages ont été criblés à l'aide de l'ADNc isolé chez *Eucalyptus gunnii* (fragment Xhol de 804 pb de pEUCCR) marqué par random priming au dCTPα³²p.

Les conditions d'hybridation utilisées pour le criblage sont les suivantes: la préhybridation est effectuée dans 0,25% Marvel, 5X SSPE, 0,05% SDS à 58°C. L'hybridation est réalisée dans ce même tampon, en présence de 50 ng de sonde marquée au dCTPα³²p, à 58°C. Les filtres sont alors lavés par 2X SSC/0,1% SDS pendant 20 minutes à température ambiante, 2X SSC/0, 1% SDS pendant 20 minutes à 58°C, 1X SSC/0,1% SDS pendant 15 minutes à 58°C.

Le plasmide pBluescript SK⁻ contenant le clone d'ADNc (cloné dans le site EcoR1, en utilisant des adaptateurs EcoR1), a été excisé *in vivo*.

L'ADNc obtenu est représenté par SEQ ID NO 7, et la séquence en acides aminés déduite de cet ADNc est représentée par SEQ ID NO 8.

## Revendications

1. Utilisation de séquences nucléotidiques recombinantes contenant une ou plusieurs région(s) codante(s), cette (ces) région(s) codante(s) étant constituée(s) :
a) d'une séquence nucléotidique codant pour un ARN messager (ARNm), cet ARNm codant lui-même pour une cinnamoyl CoA réductase (CCR) chez les plantes, ou d'une séquence nucléotidique dérivée de la séquence nucléotidique susmentionnée, notamment par mutation et/ou addition, et/ou suppression et/ou substitution d'un ou plusieurs nucléotides, cette séquence dérivée codant pour un ARNm, cet ARNm codant lui-même pour une protéine dérivée présentant une activité CCR, ou
b) d'une séquence nucléotidique complémentaire de la séquence nucléotidique codant pour un ARNm, ou de la séquence dérivée de cette dernière, tels que définies en a), cette séquence complémentaire codant pour un ARNm antisens susceptible de s'hybrider avec l'un des ARNm susmentionnés, ladite **hybridation ayant pour effet d'inhiber la synthèse de la CCR**
pour la transformation de cellules végétales, en vue de l'obtention de plantes transgéniques au sein desquelles la synthèse de lignine est régulée soit dans le sens d'une augmentation, soit dans le sens d'une diminution, des teneurs en lignine produites, par rapport aux teneurs normales en lignine produites chez les plantes.

2. Utilisation de séquences nucléotidiques recombinantes selon la revendication 1, **caractérisée en ce qu'**elles contiennent, à titre de région codante, tout ou partie d'une séquence nucléotidique choisie parmi les suivantes :
- la séquence nucléotidique représentée par SEQ ID N° 1, codant pour un ARNm, cet ARNm codant lui-même pour la CCR d'eucalyptus représentée par SEQ ID N° 2,
- la séquence nucléotidique représentée par SEQ ID N° 3, codant pour un ARNm, cet ARNm codant lui-même pour la CCR de peuplier représentée par SEQ ID N° 4,
- la séquence nucléotidique représentée par SEQ ID N° 5, codant pour un ARNm, cet ARNm codant lui-même pour la CCR de fétuque représentée par SEQ ID N° 6,
- la séquence nucléotidique représentée par SEQ ID N° 7, codant pour un ARNm, cet ARNm codant lui-même pour la CCR de tabac représentée par SEQ ID N° 8,
- la séquence nucléotidique complémentaire de celle représentée par SEQ ID N° 1, SEQ ID N° 3, SEQ ID N° 5, ou SEQ ID N° 7, cette séquence complémentaire codant pour un ARNm antisens susceptible de s'hybrider avec l'ARNm codé par les séquences SEQ ID N° 1, SEQ ID N° 3, SEQ ID N° 5, ou SEQ ID N° 7 **respectivement ladite hybridation ayant pour effet d'inhiber la synthèse de la CCR**
- la séquence nucléotidique dérivée de la séquence représentée par SEQ ID N° 1, SEQ ID N° 3, SEQ ID N° 5, ou SEQ ID N° 7, notamment par mutation et/ou addition, et/ou suppression et/ou substitution d'un ou plusieurs nucléotides, cette séquence dérivée codant soit pour un ARNm codant lui-même pour la CCR représentée par SEQ ID N° 2, SEQ ID N° 4, SEQ ID N° 6, ou SEQ ID N° 8 respectivement, ou pour une protéine dérivée de ces dernières et présentant une activité CCR chez les plantes, notamment la séquence dérivée de la séquence SEQ ID N° 1, et représentée par SEQ ID N° 9, cette dernière codant pour un ARNm codant lui-même pour une protéine représentée par SEQ ID N° 10 dérivée de la CCR d'eucalyptus susmentionnée,
- la séquence nucléotidique dérivée de la séquence nucléotidique complémentaire susmentionnée, par mutation et/ou addition, et/ou suppression et/ou substitution d'un ou plusieurs nucléotides, cette séquence dérivée codant pour un ARNm antisens susceptible de s'hybrider avec un des ARNm susmentionnés, **ladite hybridation ayant pour effet d'inhiber la synthèse de la CCR**

3. Séquence D'ADN, **caractérisée en ce qu'**elle comprend, à titre de région codante :
- la séquence nucléotidique représentée par SEQ ID N° 1, codant pour un ARNm, cet ARNm codant lui-même pour la CCR représentée par SEQ ID N° 2, ou
- la séquence nucléotidique dérivée de la SEQ ID N° 1, et représentée par SEQ ID N° 9, cette dernière codant pour un ARNm, cet ARNm codant lui-même pour une protéine représentée par SEQ ID N° 10 dérivée de la CCR d'eucalyptus.

4. Séquence D'ADN, **caractérisée en ce qu'**elle comprend, à titre de région codante, la séquence nucléotidique représentée par SEQ ID N° 3, codant pour un ARNm, cet ARNm codant lui-même pour la CCR représentée par SEQ ID N° 4.

5. Séquence D'ADN, **caractérisée en ce qu'**elle comprend, à titre de région codante, la séquence nucléotidique représentée par SEQ ID N° 5 codant pour un ARNm, cet ARNm codant lui-même pour la CCR représentée par SEQ ID N° 6.

6. Séquence D'ADN, **caractérisée en ce qu'**elle comprend, à titre de région codante, la séquence nucléotidique représentée par SEQ ID N° 7 codant pour un ARNm, cet ARNm codant lui-même pour la CCR représentée par SEQ ID N° 8.

7. Séquence D'ADN, **caractérisée en ce qu'**elle comprend, à titre de région codante la séquence nucléotidique complémentaire de celle représentée par SEQ ID N° 1, cette séquence complémentaire codant pour un ARNm antisens susceptible de s'hybrider avec l'ARNm codant lui-même pour la CCR représentée par SEQ ID N° 2, à savoir l'ARNm codé par la séquence représentée par SEQ ID N° 1, ou la séquence nucléotidique complémentaire de celle représentée par SEQ ID N° 9, cette séquence codant pour un ARNm antisens susceptible de s'hybrider avec l'ARNm susmentionné, **ladite hybridation ayant pour effet d'inhiber la synthèse de la CCR**

8. Séquence D'ADN, **caractérisée en ce qu'**elle comprend, à titre de région codante la séquence nucléotidique complémentaire de celle représentée par SEQ ID N° 3, cette séquence complémentaire codant pour un ARNm antisens susceptible de s'hybrider avec l'ARNm codant lui-même pour la CCR représentée par SEQ ID N° 4, à savoir l'ARNm codé par la séquence représentée par SEQ ID N° 3, **ladite hybridation ayant pour effet d'inhiber la synthèse de la CCR.**

9. Séquence D'ADN, **caractérisée en ce qu'**elle comprend, à titre de région codante la séquence nucléotidique complémentaire de celle représentée par SEQ ID N° 5, cette séquence complémentaire codant pour un ARNm antisens susceptible de s'hybrider avec l'ARNm codant lui-même pour la CCR représentée par SEQ ID N° 6, à savoir l'ARNm codé par la séquence représentée par SEQ ID N° 5, **ladite hybridation ayant pour effet d'inhiber la synthèse de la CCR.**

10. Séquence D'ADN, **caractérisée en ce qu'**elle comprend, à titre de région codante la séquence nucléotidique complémentaire de celle représentée par SEQ ID N° 7, cette séquence complémentaire codant pour un ARNm antiscns susceptible de s'hybrider avec l'ARNm codant lui-même pour la CCR représentée par SEQ ID N° 8, à savoir l'ARNm codé par la séquence représentée par SEQ ID N° 7, **ladite hybridation ayant pour effet d'inhiber la synthèse de la CCR**

11. ARNm codé par une séquence d'ADN selon l'une des revendications 3 à 10 et plus particulièrement :
- l'ARNm codé par la séquence d'ADN représentée par SEQ ID N° 1, ledit ARNm étant susceptible de coder à son tour pour la CCR présente chez l'eucalyptus, telle que représentée par SEQ ID N° 2,
- l'ARNm codé par la séquence d'ADN représentée par SEQ ID N° 9, ledit ARNm étant susceptible de coder à son tour pour une protéine dérivé de la CC d'eucalyptus, telle que représentée par SEQ ID N° 10,
- l'ARNm codé par la séquence d'ADN représentée par SEQ ID N° 3, ledit ARNm étant susceptible de coder à son tour pour la CCR présente chez le peuplier, telle que représentée par SEQ ID N° 4,
- l'ARNm codé par la séquence d'ADN représentée par SEQ ID N° 5, ledit ARNm étant susceptible de coder à son tour pour la CCR présente chez la fétuque, telle que représentée par SEQ ID N° 6,
- l'ARNm codé par la séquence d'ADN représentée par SEQ ID N° 7, ledit ARNm étant susceptible de coder à son tour pour la CCR présente chez le tabac, telle que représentée par SEQ ID N° 8.

12. ARNm antisens, **caractérisé en ce qu'**il comprend des nucléotides complémentaires des nucléotides constituant un ARNm selon la revendication 11, et **en ce qu'**il est susceptible de s'hybrider avec ce dernier, **ladite hybridation ayant pour effet d'inhiber la synthèse de la CCR.**

13. Complexe formé entre un ARNm antisens selon la revendication 12, et un ARNm selon la revendication 11.

14. Séquence nucléotidique recombinante, **caractérisée en ce qu'**elle comprend au moins une séquence d'ADN selon l'une des revendications 3 à 6, susceptible de coder pour un ARNm lui-même susceptible de coder pour une CCR chez les plantes, ladite séquence selon l'une des revendications 3 à 6 étant insérée dans une séquence hétérologue.

15. Séquence nucléotidique recombinante, **caractérisée en ce qu'**elle comprend au moins une séquence d'ADN complémentaire selon l'une des revendications 7 à 10, insérée dans une séquence hétérologue, ladite séquence d'ADN complémentaire codant pour un ARNm antisens susceptible de s'hybrider avec l'ARNm codant pour une CCR chez les plantes, ladite hybridation ayant pour effet d'inhiber la synthèse de la CCR.

16. Séquence nucléotidique selon la revendication 14 ou la revendication 15, **caractérisée en ce qu'**elle comprend les éléments nécessaires pour réguler l'expression de la séquence nucléotidique selon l'une des revendications 3 à 6, de sa séquence complémentaire scion l'une des revendications 7 à 10, notamment un promoteur et un terminateur de la transcription de ces séquences, et le cas échant au moins une séquence d'ADN codant pour tout ou partie d'une autre enzyme que la CCR, qui se trouve être impliquée dans une étape de biosynthèse des lignines chez les plantes, notamment l'ARNm codant lui-même pour l'alcool cinnamylique désydrogénase (CAD), ou au moins une séquence codant pour tout ou partie de l'ARNm antisens susceptible de s'hybrider avec l'ARNm susmentionné, notamment avec l'ARNm codant pour la CAD.

17. Vecteur recombinant **caractérisé en ce qu'**il comprend une séquence nucléotidique recombinante selon l'une des revendications 14 à 16, intégrée dans l'un de ses sites de son génome non essentiels pour sa réplication.

18. Procédé de régulation de la biosynthèse des lignines chez les plantes, soit par diminution, soit par augmentation des teneurs en lignines produites, par rapport aux teneurs normales en lignines produites chez des plantes, ledit procédé comprenant une étape de transformation de cellules de ces plantes à l'aide d'un vecteur contenant :
a) une séquence nucléotidique codant pour un ARN messager (ARNm), cet ARNm codant lui-même pour une CCR chez les plantes, ou une séquence nucléotidique dérivée de la séquence nucléotidique susmentionnée, notamment par mutation et/ou addition, et/ou suppression et/ou substitution d'un ou plusieurs nucléotides, cette séquence dérivée codant pour un ARNm, cet ARNm codant lui-même pour une protéine dérivée présentant une activité CCR, ou
b) une séquence nucléotidique complémentaire de la séquence nucléotidique codant pour un ARNm, ou de la séquence dérivée de cette dernière, tels que définies en a), cette séquence complémentaire codant pour un ARNm antisens susceptible de s'hybrider avec l'un des ARNm susmentionnés, **ladite hybridation ayant pour effet d'inhiber la synthèse de la CCR,**
notamment à l'aide d'un vecteur selon la revendication 17.

19. Procédé de diminution de la biosynthèse des lignines chez les plantes, et donc de diminution des teneurs en lignines produites par rapport aux teneurs normales en lignines produites chez les plantes, **caractérisée en ce qu'**il est effectué par transformation du génome de ces plantes, en y incorporant :
- au moins une séquence d'ADN selon l'une des revendications 7 à 10,
- et, le cas échéant, au moins une séquence d'ADN codant pour une autre enzyme que la CCR, qui se trouve être impliquée dans une étape de la biosynthèse des lignines chez les plantes, notamment l'ARNm codant pour la CAD
ladite transformation étant réalisée :
- soit à l'aide d'un vecteur recombinant selon la revendication 17, contenant une séquence nucléotidique recombinante selon la revendication 15 ou la revendication 16,
- soit à l'aide de plusieurs vecteurs recombinants dont l'un au moins contient une séquence nucléotidique recombinante selon la revendication 15, tandis que l'(ou les) autre(s) vecteur(s) recombinant(s) contien(nen)t une séquence d'ADN codant pour un ARNm antisens capable de s'hybrider à un ARNm codant pour une autre enzyme que la CCR, telle que définie ci-dessus.

20. Procédé de diminution de la biosynthèse des lignines chez les plantes, et donc de diminution des teneurs en lignines produites par rapport aux teneurs normales en lignines produites chez les plantes, **caractérisée en ce qu'**il est effectué par transformation du génome de ces plantes, en y incorporant :
- au moins une séquence d'ADN selon l'une des revendications 3 à 6,
- et, le cas échéant, au moins une séquence d'ADN codant pour tout ou partie d'une autre enzyme que la CCR, qui se trouve être impliquée dans une étape de la biosynthèse des lignines chez les plantes, notamment une séquence d'ADN codant pour tout ou partie de la CAD
ladite transformation étant réalisée :
- soit à l'aide d'un vecteur recombinant selon la revendication 17, contenant une séquence nucléotidique recombinante selon la revendication 14 ou la revendication 16,
- soit à l'aide de plusieurs vecteurs recombinants dont l'un au moins contient une séquence nucléotidique recombinante selon la revendication 14, tandis que l'(ou les) autre(s) vecteur(s) recombinant(s) contien(nen)t une séquence d'ADN codant pour tout ou partie d'une enzyme autre que la CCR, telle que définie ci-dessus.

21. Procédé d'augmentation de la biosynthèse des lignines chez les plantes, et donc d'augmentation des teneurs en lignines produites par rapport aux teneurs normales en lignines produites chez les plantes, **caractérisée en ce qu'**il est effectué par transformation du génome de ces plantes, en y incorporant :
- au moins une séquence d'ADN selon l'une des revendications 3 à 6,
- et, le cas échéant, au moins une séquence d'ADN codant une autre enzyme que la CCR, qui se trouve être impliquée dans une étape de la biosynthèse des lignines chez les plantes, notamment une séquence d'ADN codant pour la CAD
ladite transformation étant réalisée :
- soit à l'aide d'un vecteur recombinant selon la revendication 17, contenant une séquence nucléotidique recombinante selon la revendication 14 ou la revendication 16,
- soit à l'aide de plusieurs vecteurs recombinants dont l'un au moins contient une séquence nucléotidique recombinante selon la revendication 14, tandis que l'(ou les) autre(s) vecteur(s) recombinant(s) contien(nen)t une séquence d'ADN codant pour tout ou partie d'une enzyme autre que la CCR, telle que définie ci-dessus.

22. Plantes ou fragments de plantes, notamment cellules, fruits, semences, pollen, transformés par incorporation, dans leur génome, d'au moins une séquence nucléotidique selon l'une des revendications 3 à 10.

23. Polypeptides recombinants, notamment CCR recombinantes représentées par SEQ ID N° 2 ; SEQ ID N° 4, SEQ ID N° 6, ou SEQ ID N° 8, tels qu'obtenus par transformation de cellules végétales en intégrant de façon stable dans leur génome, une séquence nucléotidique recombinante selon l'une des revendications 14 à 16, notamment à l'aide d'un vecteur selon la revendication 17.

## Patentansprüche

1. Verwendung von rekombinanten Nukleotidsequenzen, die eine oder mehrere Kodierregion(en) enthalten, wobei diese Kodierregion(en) folgendermaßen zusammengesetzt ist/sind:
a) aus einer Nukleotidsequenz, die für eine Boten-RNA (mRNA) kodiert, wobei diese mRNA wiederum für eine Cinnamoyl-CoA-Reduktase (CCR) in Pflanzen kodiert, oder einer Nukleotidsequenz, die sich von der obengenannten Nukleotidsequenz insbesondere durch Mutation und/oder Addition, und/oder Suppression und/oder Substitution von einem oder mehreren Nukleotiden ableitet, wobei diese abgeleitete Sequenz für eine mRNA kodiert, wobei diese mRNA wiederum für ein abgeleitetes Protein, das CCR-Aktivität aufweist, kodiert, oder
b) aus einer Nukleotidsequenz, die zu der für eine mRNA kodierenden Nukleotidsequenz oder zu der von letzterer abgeleiteten Sequenz wie in a) definiert komplementär ist, wobei die komplementäre Sequenz für eine Antisense-mRNA, die mit einer der obengenannten mRNAs zu hybridisieren vermag, kodiert, wobei sich diese Hybridisierung dahingehend auswirkt, daß die CCR-Synthese gehemmt wird,
für die Transformation von pflanzlichen Zellen zwecks Gewinnung von transgenen Pflanzen, in denen die Ligninsynthese im Vergleich zu den normalen von den Pflanzen produzierten Ligningehalten entweder in Form einer Erhöhung oder in Form einer Erniedrigung der produzierten Ligningehalte reguliert ist.

2. Verwendung von rekombinanten Nukleotidsequenzen nach Anspruch 1, **dadurch gekennzeichnet, daß** sie als Kodierregion die Gesamtheit oder einen Teil einer Nukleotidsequenz, die aus den folgenden Sequenzen ausgewählt ist, enthalten:
- die Nukleotidsequenz gemäß SEQ ID Nr. 1, die für eine mRNA kodiert, wobei die mRNA wiederum für die Eukalyptus-CCR gemäß SEQ ID Nr. 2 kodiert,
- die Nukleotidsequenz gemäß SEQ ID Nr. 3, die für eine mRNA kodiert, wobei die mRNA wiederum für die Pappel-CCR gemäß SEQ ID Nr. 4 kodiert,
- die Nukleotidsequenz gemäß SEQ ID Nr. 5, die für eine mRNA kodiert, wobei die mRNA wiederum für die Schwingel-CCR gemäß SEQ ID Nr. 6 kodiert,
- die Nukleotidsequenz gemäß SEQ ID Nr. 7, die für eine mRNA kodiert, wobei die mRNA wiederum für die Tabak-CCR gemäß SEQ ID Nr. 8 kodiert,
- die zu der Nukleotidsequenz gemäß SEQ ID Nr. 1, SEQ ID Nr. 3, SEQ ID Nr. 5 oder SEQ ID Nr. 7 komplementäre Nukleotidsequenz, wobei die Komplementärsequenz für eine Antisense-mRNA, die mit der von den Sequenzen SEQ ID Nr. 1, SEQ ID Nr. 3, SEQ ID Nr. 5 bzw. SEQ ID Nr. 7 kodierten mRNA zu hybridisieren vermag, kodiert, wobei sich diese Hybridisierung dahingehend auswirkt, daß die CCR-Synthese gehemmt wird,
- die von der Sequenz gemäß SEQ ID Nr. 1, SEQ ID Nr. 3, SEQ ID Nr. 5 oder SEQ ID Nr. 7 insbesondere durch Mutation und/oder Addition, und/oder Suppression und/oder Substitution von einem oder mehreren Nukleotiden abgeleitete Nukleotidsequenz, wobei diese abgeleitete Sequenz entweder für eine mRNA, die selbst wiederum für die CCR gemäß SEQ ID Nr. 2, SEQ ID Nr. 4, SEQ ID Nr. 6 bzw. SEQ ID Nr. 8 kodiert, oder für ein von letzteren abgeleitetes Protein, das eine CCR-Aktivität in Pflanzen aufweist, kodiert, insbesondere die von SEQ ID Nr. 1 abgeleitete Sequenz gemäß SEQ ID Nr. 9, wobei letztere für eine mRNA kodiert, die selbst wiederum für ein Protein gemäß SEQ ID Nr. 10 kodiert, welches von der obengenannten Eukalyptus-CCR abgeleitet ist,
- die Nukleotidsequenz, die von der obengenannten komplementären Nukleotidsequenz durch Mutation und/oder Addition und/oder Suppression und/oder Substitution von einem oder mehreren Nukleotiden abgeleitet ist, wobei diese abgeleitete Sequenz für eine Antisense-mRNA kodiert, die mit einer der obengenannten mRNAs zu hybridisieren vermag, wobei sich diese Hybridisierung dahingehend auswirkt, daß die CCR-Synthese gehemmt wird.

3. DNA-Sequenz, **dadurch gekennzeichnet, daß** sie als Kodierregion folgendes umfaßt:
- die Nukleotidsequenz gemäß SEQ ID Nr. 1, die für eine mRNA kodiert, wobei die mRNA wiederum für die CCR gemäß SEQ ID Nr. 2 kodiert, oder
- die von SEQ ID Nr. 1 abgeleitete Nukleotidsequenz gemäß SEQ ID Nr. 9, wobei letztere für eine mRNA kodiert, wobei die mRNA wiederum für ein von der Eukalyptus-CCR abgeleitetes Protein gemäß SEQ ID Nr. 10 kodiert.

4. DNA-Sequenz, **dadurch gekennzeichnet, daß** sie als Kodierregion die Nukleotidsequenz gemäß SEQ ID Nr. 3, die für eine mRNA kodiert, wobei diese mRNA wiederum für die CCR gemäß SEQ ID Nr. 4 kodiert, enthält.

5. DNA-Sequenz, **dadurch gekennzeichnet, daß** sie als Kodierregion die Nukleotidsequenz gemäß SEQ ID Nr. 5, die für eine mRNA kodiert, wobei diese mRNA wiederum für die CCR gemäß SEQ ID Nr. 6 kodiert, enthält.

6. DNA-Sequenz, **dadurch gekennzeichnet, daß** sie als Kodierregion die Nukleotidsequenz gemäß SEQ ID Nr. 7, die für eine mRNA kodiert, wobei diese mRNA wiederum für die CCR gemäß SEQ ID Nr. 8 kodiert, enthält.

7. DNA-Sequenz, **dadurch gekennzeichnet, daß** sie als Kodierregion die Nukleotidsequenz, die zu der Sequenz gemäß SEQ ID Nr. 1 komplementär ist, wobei diese komplementäre Sequenz für eine Antisense-mRNA kodiert, die mit der mRNA, die selbst wiederum für die CCR gemäß SEQ ID Nr. 2 kodiert, nämlich der mRNA, die von der Sequenz gemäß SEQ ID Nr. 1 kodiert wird, zu hybridisieren vermag, oder die Nukleotidsequenz, die zu der Sequenz gemäß SEQ ID Nr. 9 komplementär ist, wobei diese Sequenz für eine Antisense-mRNA kodiert, die mit der obengenannten mRNA zu hybridisieren vermag, wobei sich diese Hybridisierung dahingehend auswirkt, daß die CCR-Synthese gehemmt wird, enthält.

8. DNA-Sequenz, **dadurch gekennzeichnet, daß** sie als Kodierregion die Nukleotidsequenz, die zu der Sequenz gemäß SEQ ID Nr. 3 komplementär ist, enthält, wobei diese komplementäre Sequenz für eine Antisense-mRNA kodiert, die mit der mRNA, die selbst wiederum für die CCR gemäß SEQ ID Nr. 4 kodiert, nämlich der mRNA, die von der Sequenz gemäß SEQ ID Nr. 3 kodiert wird, zu hybridisieren vermag, wobei sich diese Hybridisierung dahingehend auswirkt, daß die CCR-Synthese gehemmt wird.

9. DNA-Sequenz, **dadurch gekennzeichnet, daß** sie als Kodierregion die Nukleotidsequenz, die zu der Sequenz gemäß SEQ ID Nr. 5 komplementär ist, enthält, wobei diese komplementäre Sequenz für eine Antisense-mRNA kodiert, die mit der mRNA, die selbst wiederum für die CCR gemäß SEQ ID Nr. 6 kodiert, nämlich der mRNA, die von der Sequenz gemäß SEQ ID Nr. 5 kodiert wird, zu hybridisieren vermag, wobei sich diese Hybridisierung dahingehend auswirkt, daß die CCR-Synthese gehemmt wird.

10. DNA-Sequenz, **dadurch gekennzeichnet, daß** sie als Kodierregion die Nukleotidsequenz, die zu der Sequenz gemäß SEQ ID Nr. 7 komplementär ist, enthält, wobei diese komplementäre Sequenz für eine Antisense-mRNA kodiert, die mit der mRNA, die selbst wiederum für die CCR gemäß SEQ ID Nr. 8 kodiert, nämlich der mRNA, die von der Sequenz gemäß SEQ ID Nr. 7 kodiert wird, zu hybridisieren vermag, wobei sich diese Hybridisierung dahingehend auswirkt, daß die CCR-Synthese gehemmt wird.

11. mRNA, die von einer DNA-Sequenz nach einem der Ansprüche 3 bis 10 kodiert wird, insbesondere:
- die mRNA, die von der DNA-Sequenz gemäß SEQ ID Nr. 1 kodiert wird, wobei diese mRNA wiederum für die beim Eukalyptus vorhandene CCR gemäß SEQ ID Nr. 2 zu kodieren vermag,
- die mRNA, die von der DNA-Sequenz gemäß SEQ ID Nr. 9 kodierte mRNA, wobei die mRNA wiederum für ein von der Eukalyptus-CCR abgeleitetes Protein gemäß SEQ ID Nr. 10 zu kodieren vermag,
- die mRNA, die von der DNA-Sequenz gemäß SEQ ID Nr. 3 kodiert wird, wobei diese mRNA wiederum für die in der Pappel vorhandene CCR gemäß SEQ ID Nr. 4 zu kodieren vermag,
- die mRNA, die von der DNA-Sequenz gemäß SEQ ID Nr. 5 kodiert wird, wobei diese mRNA wiederum für die beim Schwingel vorhandene CCR gemäß SEQ ID Nr. 6 zu kodieren vermag,
- die mRNA, die von der DNA-Sequenz gemäß SEQ ID Nr. 7 kodiert wird, wobei diese mRNA wiederum für die beim Tabak vorhandene CCR gemäß SEQ ID Nr. 8 zu kodieren vermag.

12. Antisense-mRNA, **dadurch gekennzeichnet, daß** sie Nukleotide enthält, die zu Nukleotiden komplementär sind, welche eine mRNA gemäß Anspruch 11 darstellen, und dadurch, daß sie mit letzterer zu hybridisieren vermag, wobei sich diese Hybridisierung dahingehend auswirkt, daß die CCR-Synthese gehemmt wird.

13. Komplex, der zwischen einer Antisense-mRNA gemäß Anspruch 12 und einer mRNA gemäß Anspruch 11 gebildet wird.

14. Rekombinante Nukleotidsequenz, **dadurch gekennzeichnet, daß** sie mindestens eine DNA-Sequenz nach einem der Ansprüche 3 bis 6 enthält, die für eine mRNA zu kodieren vermag, die selbst wiederum für eine CCR bei Pflanzen zu kodieren vermag, wobei die Sequenz nach einem der Ansprüche 3 bis 6 in eine heterologe Sequenz insertiert ist.

15. Rekombinante Nukleotidsequenz, **dadurch gekennzeichnet, daß** sie mindestens eine komplementäre DNA-Sequenz nach einem der Ansprüche 7 bis 10 enthält, die in eine heterologe Sequenz insertiert ist, wobei die komplementäre DNA-Sequenz für eine Antisense-mRNA kodiert, die mit der mRNA, die für eine CCR bei Pflanzen kodiert, zu hybridisieren vermag, wobei sich diese Hybridisierung dahingehend auswirkt, daß die CCR-Synthese gehemmt wird.

16. Nukleotidsequenz nach Anspruch 14 oder 15, **dadurch gekennzeichnet, daß** sie die für die Regelung der Expression der Nukleotidsequenz nach einem der Ansprüche 3 bis 6, seiner komplementären Sequenz nach einem der Ansprüche 7 bis 10, erforderlichen Elemente, insbesondere einen Promoter und einen Terminator für die Transkription dieser Sequenzen, gegebenenfalls mindestens eine DNA-Sequenz, die für die Gesamtheit oder einen Teil eines Enzyms, bei dem es sich nicht um die CCR handelt und das an einem Ligninbiosyntheseschritt bei Pflanzen beteiligt ist, kodiert, insbesondere die mRNA, die selbst wiederum für die Cinnamylalkoholdehydrogenase (CAD) kodiert, oder mindestens eine Sequenz, die für die Gesamtheit oder einen Teil der Antisense-mRNA, die mit der obengenannten mRNA, insbesondere mit der für die CAD kodierenden mRNA, zu hybridisieren vermag, kodiert, umfaßt.

17. Rekombinanter Vektor, **dadurch gekennzeichnet, daß** er eine rekombinante Nukleotidsequenz nach einem der Ansprüche 14 bis 16 in einen der Orte seines Genoms, die nicht für seine Replikation wesentlich sind, integriert enthält.

18. Verfahren für die Regulation der Ligninbiosynthese bei Pflanzen durch Erniedrigung oder Erhöhung der produzierten Ligningehalte im Vergleich zu den normalen Ligningehalten, die von den Pflanzen produziert werden, wobei dieses Verfahren einen Schritt umfaßt, bei dem Zellen dieser Pflanzen mit Hilfe eines Vektors transformiert werden, der folgendes umfaßt:
a) eine Nukleotidsequenz, die für eine Boten-RNA (mRNA) kodiert, wobei diese mRNA wiederum für eine CCR in Pflanzen kodiert, oder eine Nukleotidsequenz, die sich von der obengenannten Nukleotidsequenz insbesondere durch Mutation und/oder Addition und/oder Suppression und/oder Substitution von einem oder mehreren Nukleotiden ableitet, wobei diese abgeleitete Sequenz für eine mRNA kodiert, wobei diese mRNA wiederum für ein abgeleitetes Protein, das CCR-Aktivität aufweist, kodiert, oder
b) eine Nukleotidsequenz, die zu der für eine mRNA kodierende Nukleotidsequenz oder zu der von letzterer abgeleiteten Sequenz wie in a) definiert komplementär ist, wobei die komplementäre Sequenz für eine Antisense-mRNA, die mit einer der obengenannten mRNAs zu hybridisieren vermag, kodiert, wobei sich diese Hybridisierung dahingehend auswirkt, daß die CCR-Synthese gehemmt wird,
insbesondere mit Hilfe eines Vektors nach Anspruch 17.

19. Verfahren zur Erniedrigung der Ligninbiosynthese bei Pflanzen und daher zur Erniedrigung der im Vergleich zu den normalen von den Pflanzen produzierten Ligningehalten produzierten Ligningehalte, **dadurch gekennzeichnet, daß** es durch Transformation des Genoms dieser Pflanzen durchgeführt wird, wobei folgendes in diese eingebracht wird:
- mindestens eine DNA-Sequenz nach einem der Ansprüche 7 bis 10,
- und gegebenenfalls mindestens eine DNA-Sequenz, die für ein Enzym, bei dem es sich nicht um die CCR handelt und das an einem Ligninbiosyntheseschritt bei Pflanzen beteiligt ist, kodiert, insbesondere die mRNA, die für die CAD kodiert,
wobei diese Transformation
- entweder mit Hilfe eines rekombinanten Vektors nach Anspruch 17, der eine rekombinante Nukleotidsequenz nach Anspruch 15 oder 16 enthält,
- oder mit Hilfe von mehreren rekombinanten Vektoren, von denen mindestens einer eine rekombinante Nukleotidsequenz nach Anspruch 15 enthält, während der andere rekombinante Vektor, bzw. die anderen rekombinanten Vektoren, der bzw. die eine DNA-Sequenz enthält bzw. enthalten, die für eine Antisense-mRNA kodiert, die mit einer mRNA, die für ein Enzym, bei dem es sich nicht um die CCR wie oben definiert handelt, kodiert, zu hybridisieren vermag,
durchgeführt wird.

20. Verfahren zur Erniedrigung der Ligninbiosynthese bei Pflanzen und daher zur Erniedrigung der im Vergleich zu den normalen von den Pflanzen produzierten Ligningehalten produzierten Ligningehalte, **dadurch gekennzeichnet, daß** es durch Transformation des Genoms dieser Pflanzen durchgeführt wird, wobei folgendes in diese eingebracht wird:
- mindestens eine DNA-Sequenz nach einem der Ansprüche 3 bis 6,
- und gegebenenfalls mindestens eine DNA-Sequenz, die für die Gesamtheit oder einen Teil eines Enzyms, bei dem es sich nicht um die CCR handelt und das an einem Ligninbiosyntheseschritt bei Pflanzen beteiligt ist, kodiert, insbesondere eine DNA-Sequenz, die für die Gesamtheit oder einen Teil der CAD kodiert,
wobei diese Transformation
- entweder mit Hilfe eines rekombinanten Vektors nach Anspruch 17, der eine rekombinante Nukleotidsequenz nach Anspruch 14 oder 16 enthält,
- oder mit Hilfe von mehreren rekombinanten Vektoren, von denen mindestens einer eine rekombinante Nukleotidsequenz nach Anspruch 14 enthält, während der andere rekombinante Vektor, bzw. die anderen rekombinanten Vektoren eine DNA-Sequenz, die für die Gesamtheit oder einen Teil eines Enzyms, bei dem es sich nicht um die CCR wie oben definiert handelt, kodiert, enthält/enthalten,
durchgeführt wird.

21. Verfahren zur Erhöhung der Ligninbiosynthese bei Pflanzen und daher zur Erhöhung der im Vergleich zu den normalen von den Pflanzen produzierten Ligningehalten produzierten Ligningehalte, **dadurch gekennzeichnet, daß** es durch Transformation des Genoms dieser Pflanzen durchgeführt wird, wobei folgendes in diese eingebracht wird:
- mindestens eine DNA-Sequenz nach einem der Ansprüche 3 bis 6,
- und gegebenenfalls mindestens eine DNA-Sequenz, die für ein Enzym, bei dem es sich nicht um die CCR handelt und das an einem Ligninbiosyntheseschritt bei Pflanzen beteiligt ist, kodiert, insbesondere eine DNA-Sequenz, die für die CAD kodiert,
wobei diese Transformation
- entweder mit Hilfe eines rekombinanten Vektors nach Anspruch 17, der eine rekombinante Nukleotidsequenz nach Anspruch 14 oder 16 enthält,
- oder mit Hilfe von mehreren rekombinanten Vektoren, von denen mindestens einer eine rekombinante Nukleotidsequenz nach Anspruch 14 enthält, während der andere rekombinante Vektor, bzw. die anderen rekombinanten Vektoren eine DNA-Sequenz enthält bzw. enthalten, die für die Gesamtheit oder einen Teil eines Enzyms, bei dem es sich nicht um die CCR wie oben definiert handelt, kodiert.
durchgeführt wird.

22. Pflanzen oder Pflanzenteile, insbesondere Zellen, Früchte, Samen, Pollen, die dadurch transformiert wurden, daß man in ihr Genom mindestens eine Nukleotidsequenz nach einem der Ansprüche 3 bis 10 einbringt.

23. Rekombinante Polypeptide, insbesondere rekombinante CCR, gemäß SEQ ID Nr. 2; SEQ ID Nr. 4; SEQ ID Nr. 6 oder SEQ ID Nr. 8, die dadurch erhalten wurden, daß man pflanzliche Zellen dadurch transformiert, daß man eine rekombinante Nukleotidsequenz nach einem der Ansprüche 14 bis 16, insbesondere mit Hilfe eines Vektors nach Anspruch 17, stabil in ihr Genom einbringt.

## Claims

1. Use of recombinant nucleotide sequences containing one or more coding region(s), this(these) coding region(s) consisting:
a) of a nucleotide sequence encoding a messenger RNA (mRNA), this mRNA itself encoding a cinnamoyl CoA reductase (CCR) in plants, or of a nucleotide sequence derived from the abovementioned nucleotide sequence, in particular by mutation and/or addition and/or deletion and/or substitution of one or more nucleotides, this derived sequence encoding an mRNA, this mRNA itself encoding a derived protein exhibiting CCR activity, or
b) of a nucleotide sequence complementary to the nucleotide sequence encoding an mRNA, or to the sequence derived from the latter sequence, as defined in a), this complementary sequence encoding an antisense mRNA capable of hybridizing with one of the abovementioned mRNAs, said hybridization having the effect of inhibiting the synthesis of the CCR,
for transforming plant cells for the purpose of obtaining transgenic plants in which the synthesis of lignin is regulated either in the direction of an increase, or in the direction of a decrease, in the lignin contents produced, compared with the normal lignin contents produced in plants.

2. Use of recombinant nucleotide sequences according to Claim 1, **characterized in that** they contain, as coding region, all or part of a nucleotide sequence chosen from the following:
- the nucleotide sequence represented by SEQ ID No. 1, encoding an mRNA, this mRNA itself encoding the eucalyptus CCR represented by SEQ ID No. 2,
- the nucleotide sequence represented by SEQ ID No. 3, encoding an mRNA, this mRNA itself encoding the poplar CCR represented by SEQ ID No. 4,
- the nucleotide sequence represented by SEQ ID No. 5, encoding an mRNA, this mRNA itself encoding the fescue CCR represented by SEQ ID No. 6,
- the nucleotide sequence represented by SEQ ID No. 7, encoding an mRNA, this mRNA itself encoding the tobacco CCR represented by SEQ 10 No.8,
- the nucleotide sequence complementary to that represented by SEQ ID No. 1, SEQ ID No. 3, SEQ ID No. 5 or SEQ ID No. 7, this complementary sequence encoding an antisense mRNA capable of hybridizing with the mRNA encoded by the sequences SEQ ID No. 1, SEQ ID No. 3, SEQ ID No. 5 or SEQ ID No. 7, respectively, said hybridization having the effect of inhibiting the synthesis of the CCR,
- the nucleotide sequence derived from the sequence represented by SEQ ID No. 1, SEQ ID No. 3, SEQ ID No. 5 or SEQ ID No. 7, in particular by mutation and/or addition and/or deletion and/or substitution of one or more nucleotides, this derived sequence encoding either an mRNA itself encoding the CCR represented by SEQ ID No. 2, SEQ ID No. 4, SEQ ID No. 6 or SEQ ID No. 8, respectively, or a protein derived from the latter and exhibiting CCR activity in plants, in particular the sequence derived from the sequence SEQ ID No. 1, and represented by SEQ ID No. 9, the latter encoding an mRNA itself encoding a protein represented by SEQ ID No. 10 derived from the abovementioned eucalyptus CCR,
- the nucleotide sequence derived from the abovementioned complementary nucleotide sequence, by mutation and/or addition and/or deletion and/or substitution of one or more nucleotides, this derived sequence encoding an antisense mRNA capable of hybridizing with one of the abovementioned mRNAs, said hybridization having the effect of inhibiting the synthesis of the CCR.

3. DNA sequence, **characterized in that** it comprises, as coding region:
- the nucleotide sequence represented by SEQ ID No. 1, encoding an mRNA, this mRNA itself encoding the CCR represented by SEQ ID No. 2, or
- the nucleotide sequence derived from SEQ ID No. 1, and represented by SEQ ID No. 9, the latter encoding an mRNA, this mRNA itself encoding a protein represented by SEQ ID No. 10 derived from the eucalyptus CCR.

4. DNA sequence, **characterized in that** it comprises, as coding region, the nucleotide sequence represented by SEQ ID No. 3, encoding an mRNA, this mRNA itself encoding the CCR represented by SEQ ID No. 4.

5. DNA sequence, **characterized in that** it comprises, as coding region, the nucleotide sequence represented by SEQ ID No. 5, encoding an mRNA, this mRNA itself encoding the CCR represented by SEQ ID No. 6.

6. DNA sequence, **characterized in that** it comprises, as coding region, the nucleotide sequence represented by SEQ ID No. 7, encoding an mRNA, this mRNA itself encoding the CCR represented by SEQ ID No. 8.

7. DNA sequence, **characterized in that** it comprises, as coding region, the nucleotide sequence complementary to that represented by SEQ ID No. 1, this complementary sequence encoding an antisense mRNA capable of hybridizing with the mRNA itself encoding the CCR represented by SEQ ID No. 2, namely the mRNA encoded by the sequence represented by SEQ ID No. 1, or the nucleotide sequence complementary to that represented by SEQ ID No. 9, this sequence encoding an antisense mRNA capable of hybridizing with the abovementioned mRNA, said hybridization having the effect of inhibiting the synthesis of the CCR.

8. DNA sequence, **characterized in that** it comprises, as coding region, the nucleotide sequence complementary to that represented by SEQ ID No. 3, this complementary sequence encoding an antisense mRNA capable of hybridizing with the mRNA itself encoding the CCR represented by SEQ ID No. 4, namely the mRNA encoded by the sequence represented by SEQ ID No. 3, said hybridization having the effect of inhibiting the synthesis of the CCR.

9. DNA sequence, **characterized in that** it comprises, as coding region, the nucleotide sequence complementary to that represented by SEQ ID No. 5, this complementary sequence encoding an antisense mRNA capable of hybridizing with the mRNA itself encoding the CCR represented by SEQ ID No. 6, namely the mRNA encoded by the sequence represented by SEQ ID No. 5, said hybridization having the effect of inhibiting the synthesis of the CCR.

10. DNA sequence, **characterized in that** it comprises, as coding region, the nucleotide sequence complementary to that represented by SEQ ID No. 7, this complementary sequence encoding an antisense mRNA capable of hybridizing with the mRNA itself encoding the CCR represented by SEQ ID No. 8, namely the mRNA encoded by the sequence represented by SEQ ID No. 7, said hybridization having the effect of inhibiting the synthesis of the CCR.

11. mRNA encoded by a DNA sequence according to one of Claims 3 to 10 and more particularly:
- the mRNA encoded by the DNA sequence represented by SEQ ID No. 1, said mRNA being capable of encoding, in turn, the CCR present in eucalyptus, as represented by SEQ ID No. 2,
- the mRNA encoded by the DNA sequence represented by SEQ ID No. 9, said mRNA being capable of encoding, in turn, a protein derived from the eucalyptus CCR, as represented by SEQ ID No. 10,
- the mRNA encoded by the DNA sequence represented by SEQ ID No. 3, said mRNA being capable of encoding, in turn, the CCR present in poplar, as represented by SEQ ID No. 4,
- the mRNA encoded by the DNA sequence represented by SEQ ID No. 5, said mRNA being capable of encoding, in turn, the CCR present in fescue, as represented by SEQ ID No. 6,
- the mRNA encoded by the DNA sequence represented by SEQ ID No. 7, said mRNA being capable of encoding, in turn, the CCR present in tobacco, as represented by SEQ ID No. 8.

12. Antisense mRNA, **characterized in that** it comprises nucleotides complementary to the nucleotides constituting an mRNA according to Claim 11, and **in that** it is capable of hybridizing with the latter, said hybridization having the effect of inhibiting the synthesis of the CCR.

13. Complex formed between an antisense mRNA according to Claim 12 and an mRNA according to Claim 11.

14. Recombinant nucleotide sequence, **characterized in that** it comprises at least one DNA sequence according to one of Claims 3 to 6, capable of encoding an mRNA itself capable of encoding a CCR in plants, said sequence according to one of Claims 3 to 6 being inserted into a heterologous sequence.

15. Recombinant nucleotide sequence, **characterized in that** it comprises at least one complementary DNA sequence according to one of Claims 7 to 10, inserted into a heterologous sequence, said complementary DNA sequence encoding an antisense mRNA capable of hybridizing with the mRNA encoding a CCR in plants, said hybridization having the effect of inhibiting the synthesis of the CCR.

16. Nucleotide sequence according to Claim 14 or Claim 15, **characterized in that** it comprises the elements required for regulating the expression of the nucleotide sequence according to one of Claims 3 to 6, or of the sequence complementary thereto according to one of Claims 7 to 10, in particular a promoter and a terminator for the transcription of these sequences, and, where appropriate, at least one DNA sequence encoding all or part of an enzyme other than CCR, which is found to be involved in a lignin biosynthesis step in plants, in particular the mRNA itself encoding cinnamyl alcohol dehydrogenase (CAD), or at least one sequence encoding all or part of the antisense mRNA capable of hybridizing with the abovementioned mRNA, in particular with the mRNA encoding CAD.

17. Recombinant vector, **characterized in that** it comprises a recombinant nucleotide sequence according to one of Claims 14 to 16, integrated into one of the sites of its genome that are not essential for its replication.

18. Method for regulating the biosynthesis of lignins in plants, either by decreasing or by increasing the lignin contents produced, compared with the normal lignin contents produced in plants, said method comprising a step for transforming cells of these plants using a vector containing:
a) a nucleotide sequence encoding a messenger RNA (mRNA), this mRNA itself encoding a CCR in plants, or a nucleotide sequence derived from the abovementioned nucleotide sequence, in particular by mutation and/or addition and/or deletion and/or substitution of one or more nucleotides, this derived sequence encoding an mRNA, this mRNA itself encoding a derived protein exhibiting CCR activity, or
b) a nucleotide sequence complementary to the nucleotide sequence encoding an mRNA, or to the sequence derived from the latter sequence, as defined in a), this complementary sequence encoding an antisense mRNA capable of hybridizing with one of the abovementioned mRNAs, said hybridization having the effect of inhibiting the synthesis of the CCR,
in particular using a vector according to Claim 17.

19. Method for decreasing the biosynthesis of lignins in plants, and therefore for decreasing the lignin contents produced compared with the normal lignin contents produced in plants, **characterized in that** it is carried out by means of transformation of the genome of these plants, by incorporating therein:
- at least one DNA sequence according to one of Claims 7 to 10,
- and, where appropriate, at least one DNA sequence encoding an enzyme other than CCR, which is found to be involved in a lignin biosynthesis step in plants, in particular the mRNA encoding CAD,
said transformation being carried out:
- either using a recombinant vector according to Claim 17, containing a recombinant nucleotide sequence according to Claim 15 or Claim 16,
- or using several recombinant vectors, at least one of which contains a recombinant nucleotide sequence according to Claim 15, whereas the other recombinant vector(s) contain(s) a DNA sequence encoding an antisense mRNA capable of hybridizing to an mRNA encoding an enzyme other than CCR, as defined above.

20. Method of decreasing the biosynthesis of lignins in plants, and therefore for decreasing the lignin contents produced compared with the normal lignin contents produced in plants, **characterized in that** it is carried out by means of transformation of the genome of these plants, by incorporating therein:
- at least one DNA sequence according to one of Claims 3 to 6,
- and, where appropriate, at least one DNA sequence encoding all or part of an enzyme other than CCR, which is found to be involved in a lignin biosynthesis step in plants, in particular a DNA sequence encoding all or part of CAD,
said transformation being carried out:
- either using a recombinant vector according to Claim 17, containing a recombinant nucleotide sequence according to Claim 14 or Claim 16,
- or using several recombinant vectors, at least one of which contains a recombinant nucleotide sequence according to Claim 14, whereas the other recombinant vector(s) contain(s) a DNA sequence encoding all or part of an enzyme other than CCR, as defined above.

21. Method for increasing the biosynthesis of lignins in plants, and therefore for increasing the lignin contents produced compared with the normal lignin contents produced in plants, **characterized in that** it is carried out by means of transformation of the genome of these plants, by incorporating therein:
- at least one DNA sequence according to one of Claims 3 to 6,
- and, where appropriate, at least one DNA sequence encoding an enzyme other than CCR, which is found to be involved in a lignin biosynthesis step in plants, in particular a DNA sequence encoding CAD,
said transformation being carried out:
- either using a recombinant vector according to Claim 17, containing a recombinant nucleotide sequence according to Claim 14 or Claim 16,
- or using several recombinant vectors, at least one of which contains a recombinant nucleotide sequence according to Claim 14, whereas the other recombinant vector(s) contain(s) a DNA sequence encoding all or part of an enzyme other than CCR, as defined above.

22. Plants or plant fragments, in particular cells, fruits, seeds, pollen, transformed by incorporation, into their genome, of at least one nucleotide sequence according to one of Claims 3 to 10.

23. Recombinant polypeptides, in particular recombinant CCRs represented by SEQ ID No. 2, SEQ ID No. 4, SEQ ID No. 6 or SEQ ID No. 8, as obtained by transformation of plant cells by stably integrating into their genome a recombinant nucleotide sequence according to one of Claims 14 to 16, in particular using a vector according to Claim 17.


### LISTE DE SEQUENCES

(1) INFORMATION GENERALE:
   (i) DEPOSANT:
      (A) NOM: CENTRE NATIONAL DE LA RECHERCHE SCIENTIFIQUE
      (B) RUE: 3, rue Michel-Ange
      (C) VILLE: PARIS 75016
      (E) PAYS: FRANCE
      (F) CODE POSTAL: F-75016
   (ii) TITRE DE L' INVENTION: SEQUENCES D'ADN CODANT POUR LA CINNAMOYL CoA REDUCTASE, ET SES APPLICATIONS DANS LE DOMAINE DE LA REGULATION DU TAUX DE LIGNINE CHEZ LES PLANTES
   (iii) NOMBRE DE SEQUENCES: 10
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 1297 paires de bases
      (B) TYPE: acide nucléique
      (C) NOMBRE DE BRINS: double
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 136..1140
   (xi) DESCRIPTION DE LA SEQUENCE: SEQ ID NO: 1:
   CGGCCGGGAC GACCCGTTCC TCTTCTTCCG GGTCACCGTC ACCATGTTAC ACAACATCTC      60
   CGGCTAAAAA AAAAAGGAAA AAAAGCGCAA CCTCCACCTC CTGAACCCCT CTCCCCCCTC     120
   GCCGGCAATC CCACC ATG CCC GTC GAC GCC CTC CCC GGT TCC GGC CAG ACC     171
                    Met Pro Val Asp Ala Leu Pro Gly Ser Gly Gln Thr
                     1               5                  10
   GTC TGC GTC ACC GGC GCC GGC GGG TTC ATC GCC TCC TGG ATT GTC AAG     219
   Val Cys Val Thr Gly Ala Gly Gly Phe Ile Ala Ser Trp Ile Val Lys
           15                  20                  25
   CTT CTC CTC GAG CGA GGC TAC ACC GTG CGA GGA ACC GTC AGG AAC CCA     267
   Leu Leu Leu Glu Arg Gly Tyr Thr Val Arg Gly Thr Val Arg Asn Pro
       30                  35                  40
   GAC GAC CCG AAG AAT GGT CAT CTG AGA GAT CTG GAA GGA GCC AGC GAG     315
   Asp Asp Pro Lys Asn Gly His Leu Arg Asp Leu Glu Gly Ala Ser Glu
   45                  50                  55                  60
   AGG CTG ACG CTG TAC AAG GGT GAT CTG ATG GAC TAC GGG AGC TTG GAA     363
   Arg Leu Thr Leu Tyr Lys Gly Asp Leu Met Asp Tyr Gly Ser Leu Glu
                   65                  70                  75
   GAA GCC ATC AAG GGG TGC GAC GGC GTC GTC CAC ACC GCC TCT CCG GTC     411
   Glu Ala Ile Lys Gly Cys Asp Gly Val Val His Thr Ala Ser Pro Val
               80                  85                  90
   ACC GAC GAT CCT GAG CAA ATG GTG GAG CCA GCG GTG ATC GGG ACG AAA     459
   Thr Asp Asp Pro Glu Gln Met Val Glu Pro Ala Val Ile Gly Thr Lys
           95                  100                 105
   AAT GTG ATC GTC GCA GCG GCG GAG GCC AAG GTC CGG CGG GTT GTG TTC     507
   Asn Val Ile Val Ala Ala Ala Glu Ala Lys Val Arg Arg Val Val Phe
       110                 115                 120
   ACC TCC TCC ATC GGT GCA GTC ACC ATG GAC CCC AAC CGG GCA GAC GTT 555
   Thr Ser Ser Ile Gly Ala Val Thr Met Asp Pro Asn Arg Ala Asp Val
   125 130 135 140
   GTG GTG GAC GAG TCT TGT TGG AGC GAC CTC GAA TTT TGC AAG AGC ACT 603
   Val Val Asp Glu Ser Cys Trp Ser Asp Leu Glu Phe Cys Lys Ser Thr
   145 150 155
   AAG AAC TGG TAT TGC TAC GGC AAG GCA GTG GCG GAG AAG GCC GCT TGG 651
   Lys Asn Trp Tyr Cys Tyr Gly Lys Ala Val Ala Glu Lys Ala Ala Trp
   160 165 170
   CCA GAG GGC AAG GAG AGA GGG GTT GAC CTC GTG GTG ATT AAC CCT GTG 699
   Pro Glu Gly Lys Glu Arg Gly Val Asp Leu Val Val Ile Asn Pro Val
   175 180 185
   CTC GTG CTT GGA CCG CTC CTT CAG TCG ACG ATC AAT GCG AGC ATC ATC 747
   Leu Val Leu Gly Pro Leu Leu Gln Ser Thr Ile Asn Ala Ser Ile Ile
   190 195 200
   CAC ATC CTC AAG TAC TTG ACT GGC TCA GCC AAG ACC TAC GCC AAC TCG 795
   His Ile Leu Lys Tyr Leu Thr Gly Ser Ala Lys Thr Tyr Ala Asn Ser
   205 210 215 220
   GTC CAG GCG TAC GTG CAC GTC AAG GAC GTC GCG CTT GCC CAC GTC CTT 843
   Val Gln Ala Tyr Val His Val Lys Asp Val Ala Leu Ala His Val Leu
   225 230 235
   GTC TTG GAG ACC CCA TCC GCC TCA GGC CGC TAT TTG TGC GCC GAG AGC 891
   Val Leu Glu Thr Pro Ser Ala Ser Gly Arg Tyr Leu Cys Ala Glu Ser
   240 245 250
   GTC CTC CAC CGT GGC GAT GTG GTG GAA ATC CTT GCC AAG TTC TTC CCT 939
   Val Leu His Arg Gly Asp Val Val Glu Ile Leu Ala Lys Phe Phe Pro
   255 260 265
   GAG TAT AAT GTA CCG ACC AAG TGC TCT GAT GAG GTG AAC CCA AGA GTA 987
   Glu Tyr Asn Val Pro Thr Lys Cys Ser Asp Glu Val Asn Pro Arg Val
   270 275 280
   AAA CCA TAC AAG TTC TCC AAC CAG AAG CTG AGA GAC TTG GGG CTC GAG 1035
   Lys Pro Tyr Lys Phe Ser Asn Gln Lys Leu Arg Asp Leu Gly Leu Glu
   285 290 295 300
   TTC ACC CCG GTG AAG CAG TGC CTG TAC GAA ACT GTC AAG AGC TTG CAG 1083
   Phe Thr Pro Val Lys Gln Cys Leu Tyr Glu Thr Val Lys Ser Leu Gln
   305 310 315
   GAG AAA GGC CAC CTA CCA GTC CCC TCC CCG CCG GAA GAT TCG GTG CGT 1131
   Glu Lys Gly His Leu Pro Val Pro Ser Pro Pro Glu Asp Ser Val Arg
   320 325 330
   ATT CAG GGA TGATCTTAGA TCCATCACGG TGCGCATTTG TAATCCGGAG 1180
   Ile Gln Gly
   335
   AAATGAGAGA AACATGTGGG AATTTGTTTG TACTTTTCTA AGTCAAACCT GGAGATACCA 1240
   ACCCTGAGTT CTGCATTGGA ATGGAAGTTG TCAATTGTTC CAAAAAAAAA AAAAAAA 1297
(2) INFORMATION POUR LA SEQ ID NO: 2:
   (i) CARACTERISTIQUES DE LA SEQUENCE:
      (A) LONGUEUR: 335 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Pro Val Asp Ala Leu Pro Gly Ser Gly Gln Thr Val Cys Val Thr
   1 5 10 15
   Gly Ala Gly Gly Phe Ile Ala Ser Trp Ile Val Lys Leu Leu Leu Glu
   20 25 30
   Arg Gly Tyr Thr Val Arg Gly Thr Val Arg Asn Pro Asp Asp Pro Lys
   35 40 45
   Asn Gly His Leu Arg Asp Leu Glu Gly Ala Ser Glu Arg Leu Thr Leu
   50 55 60
   Tyr Lys Gly Asp Leu Met Asp Tyr Gly Ser Leu Glu Glu Ala Ile Lys
   65 70 75 80
   Gly Cys Asp Gly Val Val His Thr Ala Ser Pro Val Thr Asp Asp Pro
   85 90 95
   Glu Gln Met Val Glu Pro Ala Val Ile Gly Thr Lys Asn Val Ile Val
   100 105 110
   Ala Ala Ala Glu Ala Lys Val Arg Arg Val Val Phe Thr Ser Ser Ile
   115 120 125
   Gly Ala Val Thr Met Asp Pro Asn Arg Ala Asp Val Val Val Asp Glu
   130 135 140
   Ser Cys Trp Ser Asp Leu Glu Phe Cys Lys Ser Thr Lys Asn Trp Tyr
   145 150 155 160
   Cys Tyr Gly Lys Ala Val Ala Glu Lys Ala Ala Trp Pro Glu Gly Lys
   165 170 175
   Glu Arg Gly Val Asp Leu Val Val Ile Asn Pro Val Leu Val Leu Gly
   180 185 190
   Pro Leu Leu Gln Ser Thr Ile Asn Ala Ser Ile Ile His Ile Leu Lys
   195 200 205
   Tyr Leu Thr Gly Ser Ala Lys Thr Tyr Ala Asn Ser Val Gln Ala Tyr
   210 215 220
   Val His Val Lys Asp Val Ala Leu Ala His Val Leu Val Leu Glu Thr
   225 230 235 240
   Pro Ser Ala Ser Gly Arg Tyr Leu Cys Ala Glu Ser Val Leu His Arg
   245 250 255
   Gly Asp Val Val Glu Ile Leu Ala Lys Phe Phe Pro Glu Tyr Asn Val
   260 265 270
   Pro Thr Lys Cys Ser Asp Glu Val Asn Pro Arg Val Lys Pro Tyr Lys
   275 280 285
   Phe Ser Asn Gln Lys Leu Arg Asp Leu Gly Leu Glu Phe Thr Pro Val
   290 295 300
   Lys Gln Cys Leu Tyr Glu Thr Val Lys Ser Leu Gln Glu Lys Gly His
   305 310 315 320
   Leu Pro Val Pro Ser Pro Pro Glu Asp Ser Val Arg Ile Gln Gly
   325 330 335
(2) INFORMATION POUR LA SEQ ID NO: 3:
   (i) CARACTERISTIQUES DE LA SEQUENCE:
      (A) LONGUEUR: 1376 paires de bases
      (B) TYPE: acide nucléique
      (C) NOMBRE DE BRINS: simple
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 99..1112
   (xi) DESCRIPTION DE LA SEQUENCE: SEQ ID NO: 3:
   GAAAACACAC CTCCTCTCTT CTTTGTCTCT GTCTGTTCTC CACTTTCCCA GTCACCAAAC        60
   TCGTATGCAT ATAATTACAT TTATCTAAAT ATAACAAC ATG CCT GTT GAT GCT          113
                                             Met Pro Val Asp Ala
                                               1               5
   TCA TCA CTT TCA GGC CAA GGC CAA ACT ATC TGT GTC ACC GGG GGT GGT        161
   Ser Ser Leu Ser Gly Gln Gly Gln Thr Ile Cys Val Thr Gly Gly Gly
                    10                  15                  20
   GGT TTC ATT GCT TCT TGG ATG GTT AAA CTT CTT TTA GAT AAA GGT TAC        209
   Gly Phe Ile Ala Ser Trp Met Val Lys Leu Leu Leu Asp Lys Gly Tyr
                25                  30                  35
   ACT GTT AGA GGA ACT GCG AGG AAC CCA GCT GAT CCC AAG AAT TCT CAT        257
   Thr Val Arg Gly Thr Ala Arg Asn Pro Ala Asp Pro Lys Asn Ser His
            40                  45                  50
   TTG AGG GAG CTT GAA GGA GCT GAA GAA AGA TTA ACT TTA TGC AAA GCT        305
   Leu Arg Glu Leu Glu Gly Ala Glu Glu Arg Leu Thr Leu Cys Lys Ala
        55                  60                  65
   GAT CTT CTT GAT TAT GAG TCT CTT AAA GAG GGT ATT CAA GGG TGT GAT        353
   Asp Leu Leu Asp Tyr Glu Ser Leu Lys Glu Gly Ile Gln Gly Cys Asp
    70                  75                  80                  85
   GGT GTT TTC CAC ACT GCT TCT CCT GTC ACA GAT GAT CCG GAA GAA ATG        401
   Gly Val Phe His Thr Ala Ser Pro Val Thr Asp Asp Pro Glu Glu Met
                    90                  95                 100
   GTG GAG CCA GCA GTG AAC GGG ACC AAA AAT GTG ATA ATT GCG GCG GCT        449
   Val Glu Pro Ala Val Asn Gly Thr Lys Asn Val Ile Ile Ala Ala Ala
               105                 110                 115
   GAG GCC AAA GTC CGA CGA GTG GTG TTC ACG TCA TCA ATT GGC GCT GTG        497
   Glu Ala Lys Val Arg Arg Val Val Phe Thr Ser Ser Ile Gly Ala Val
           120                 125                 130
   TAC ATG GAT CCC AAT AAG GGC CCA GAT GTT GTC ATT GAT GAG TCT TGC        545
   Tyr Met Asp Pro Asn Lys Gly Pro Asp Val Val Ile Asp Glu Ser Cys
       135                 140                 145
   TGG AGT GAT CTT GAA TTC TGC AAG AAC ACC AAG AAT TGG TAT TGC TAT        593
   Trp Ser Asp Leu Glu Phe Cys Lys Asn Thr Lys Asn Trp Tyr Cys Tyr
   150                 155                 160                 165
   GGA AAG GCT GTG GCA GAA CAA GCT GCA TGG GAT ATG GCT AAG GAG AAA        641
   Gly Lys Ala Val Ala Glu Gln Ala Ala Trp Asp Met Ala Lys Glu Lys
                   170                 175                 180
   GGG GTG GAC CTA GTG GTG GTT AAC CCA GTG CTG GTG CTT GGA CCA TTG        689
   Gly Val Asp Leu Val Val Val Asn Pro Val Leu Val Leu Gly Pro Leu
               185                 190                 195
   TTG CAG CCC ACT GTC AAT GCT AGC ATC ACT CAC ATC CTC AAG TAC CTC        737
   Leu Gln Pro Thr Val Asn Ala Ser Ile Thr His Ile Leu Lys Tyr Leu
           200                 205                 210
   ACC GGC TCA GCC AAG ACA TAT GCT AAC TCT GTT CAA GCT TAT GTG CAT        785
   Thr Gly Ser Ala Lys Thr Tyr Ala Asn Ser Val Gln Ala Tyr Val His
       215                 220                 225
   GTT AGG GAT GTG GCA CTA GCC CAC ATT TTA GTC TTT GAG ACG CCT TCC        833
   Val Arg Asp Val Ala Leu Ala His Ile Leu Val Phe Glu Thr Pro Ser
   230                 235                 240                 245
   GCC TCC GGC CGT TAC CTT TGC TCT GAG AGC GTT CTC CAC CGT GGA GAG        881
   Ala Ser Gly Arg Tyr Leu Cys Ser Glu Ser Val Leu His Arg Gly Glu
                   250                 255                 260
   GTG GTG GAA ATC CTT GCA AAG TTC TTC CCT GAG TAC CCC ATC CCT ACC        929
   Val Val Glu Ile Leu Ala Lys Phe Phe Pro Glu Tyr Pro Ile Pro Thr
               265                 270                 275
   AAG TGC TCA GAT GAG AAG AAC CCA AGA AAA CAA CCT TAC AAG TTC TCA        977
   Lys Cys Ser Asp Glu Lys Asn Pro Arg Lys Gln Pro Tyr Lys Phe Ser
           280                 285                 290
   AAC CAG AAG CTA AGG GAT CTG GGT TTC GAA TTC ACC CCA GTA AAG CAG       1025
   Asn Gln Lys Leu Arg Asp Leu Gly Phe Glu Phe Thr Pro Val Lys Gln
       295                 300                 305
   TGT CTG TAT GAA ACT GTT AAG AGT TTG CAG GAA AAG GGT CAC CTT CCA       1073
   Cys Leu Tyr Glu Thr Val Lys Ser Leu Gln Glu Lys Gly His Leu Pro
   310                 315                 320                 325
   ATC CCA AAA CAA GCT GCA GAA GAG TCT TTG AAA ATT CAA TAAGGCCTCT        1122
   Ile Pro Lys Gln Ala Ala Glu Glu Ser Leu Lys Ile Gln
                   330                 335
   TGGAACTATT TATTAGGATT GTTCCATACC CCAAGTTTGG ATCGCAAATG CTAGGGAAAG      1182
   GAGCATATTA AAGAATGCCA ATGTGCAGGT GTTTTAGTAT TTTACATGAA GAACTCTGAT      1242
   TATCCTTGTG CTTATAATAA TTTTTTTCAA GTGAGTGTCT TCAAATGTTC AACTTGTATT      1302
   TGTGGTTGTC TAACTTTATC CAGTTTCAAT ATAAAAGAGG AACGATTCTA TGTCTTAAAA      1362
   AAAAAAAAAA AAAA                                                        1376
(2) INFORMATION POUR LA SEQ ID NO: 4:
   (i) CARACTERISTIQUES DE LA SEQUENCE:
      (A) LONGUEUR: 338 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
   Met Pro Val Asp Ala Ser Ser Leu Ser Gly Gln Gly Gln Thr Ile Cys
   1 5 10 15
   Val Thr Gly Gly Gly Gly Phe Ile Ala Ser Trp Met Val Lys Leu Leu
   20 25 30
   Leu Asp Lys Gly Tyr Thr Val Arg Gly Thr Ala Arg Asn Pro Ala Asp
   35 40 45
   Pro Lys Asn Ser His Leu Arg Glu Leu Glu Gly Ala Glu Glu Arg Leu
   50 55 60
   Thr Leu Cys Lys Ala Asp Leu Leu Asp Tyr Glu Ser Leu Lys Glu Gly
   65 70 75 80
   Ile Gln Gly Cys Asp Gly Val Phe His Thr Ala Ser Pro Val Thr Asp
   85 90 95
   Asp Pro Glu Glu Met Val Glu Pro Ala Val Asn Gly Thr Lys Asn Val
   100 105 110
   Ile Ile Ala Ala Ala Glu Ala Lys Val Arg Arg Val Val Phe Thr Ser
   115 120 125
   Ser Ile Gly Ala Val Tyr Met Asp Pro Asn Lys Gly Pro Asp Val Val
   130 135 140
   Ile Asp Glu Ser Cys Trp Ser Asp Leu Glu Phe Cys Lys Asn Thr Lys
   145 150 155 160
   Asn Trp Tyr Cys Tyr Gly Lys Ala Val Ala Glu Gln Ala Ala Trp Asp
   165 170 175
   Met Ala Lys Glu Lys Gly Val Asp Leu Val Val Val Asn Pro Val Leu
   180 185 190
   Val Leu Gly Pro Leu Leu Gln Pro Thr Val Asn Ala Ser Ile Thr His
   195 200 205
   Ile Leu Lys Tyr Leu Thr Gly Ser Ala Lys Thr Tyr Ala Asn Ser Val
   210 215 220
   Gln Ala Tyr Val His Val Arg Asp Val Ala Leu Ala His Ile Leu Val
   225 230 235 240
   Phe Glu Thr Pro Ser Ala Ser Gly Arg Tyr Leu Cys Ser Glu Ser Val
   245 250 255
   Leu His Arg Gly Glu Val Val Glu Ile Leu Ala Lys Phe Phe Pro Glu
   260 265 270
   Tyr Pro Ile Pro Thr Lys Cys Ser Asp Glu Lys Asn Pro Arg Lys Gln
   275 280 285
   Pro Tyr Lys Phe Ser Asn Gln Lys Leu Arg Asp Leu Gly Phe Glu Phe
   290 295 300
   Thr Pro Val Lys Gln Cys Leu Tyr Glu Thr Val Lys Ser Leu Gln Glu
   305 310 315 320
   Lys Gly His Leu Pro Ile Pro Lys Gln Ala Ala Glu Glu Ser Leu Lys
   325 330 335
   Ile Gln
(2) INFORMATION POUR LA SEQ ID NO: 5:
   (i) CARACTERISTIQUES DE LA SEQUENCE:
      (A) LONGUEUR: 1273 paires de bases
      (B) TYPE: acide nucléique
      (C) NOMBRE DE BRINS: simple
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 66..1091
   (xi) DESCRIPTION DE LA SEQUENCE: SEQ ID NO: 5:
   TCGTAGCTCT TCCCTTTCAC CAACAAGCTA GTTTAGACAA GTACAGTGGT ACTGTAAGAG      60
   CAACA ATG ACC GTT GTC GAC GCC GCC GCG CCG CAG CTG CCT GGC CAT         107
         Met Thr Val Val Asp Ala Ala Ala Pro Gln Leu Pro Gly His
           1               5                  10
   GGG CAG ACC GTG TGC GTC ACC GGC GCC GCG GGG TAC ATC GCG TCG GGG       155
   Gly Gln Thr Val Cys Val Thr Gly Ala Ala Gly Tyr Ile Ala Ser Gly
    15                  20                  25                  30
   CTC GTC AAG CTG CTC CTG GAG AGA GGC TAC ACC GTG AAG GGC ACA GTG       203
   Leu Val Lys Leu Leu Leu Glu Arg Gly Tyr Thr Val Lys Gly Thr Val
                    35                  40                  45
   AGG AAC CCA GAT GAT CCC AAG AAC GCC CAC CTG AAG GCG CTG GAC GGC       251
   Arg Asn Pro Asp Asp Pro Lys Asn Ala His Leu Lys Ala Leu Asp Gly
                50                  55                  60
   GCC ACC AAG AGG CTG ATC CTC TGC AAA GCC GAC CTC CTC GAC TAC GAC       299
   Ala Thr Lys Arg Leu Ile Leu Cys Lys Ala Asp Leu Leu Asp Tyr Asp
            65                  70                  75
   GCC ATA TGC GCC GCC GTC GAG GGC TGC CAC GGC GTG TTC CAC ACC GCC       347
   Ala Ile Cys Ala Ala Val Glu Gly Cys His Gly Val Phe His Thr Ala
        80                  85                  90
   TCT CCA GTC ACC GAT GAT CCT GAG CAG ATG GTG GAG CCG GCG GTG CGG    395
   Ser Pro Val Thr Asp Asp Pro Glu Gln Met Val Glu Pro Ala Val Arg
    95                 100                 105                 110
   GGC ACG GAG TAC GTG ATC AAC GCG GCA GCG GAT GCG GGA ACG GTG CGC    443
   Gly Thr Glu Tyr Val Ile Asn Ala Ala Ala Asp Ala Gly Thr Val Arg
                   115                 120                 125
   CGG GTG GTG TTC ACG TCG TCA ATC GGT GCC ATC ACC ATG GAC CCC AAC    491
   Arg Val Val Phe Thr Ser Ser Ile Gly Ala Ile Thr Met Asp Pro Asn
               130                 135                 140
   CGC GGT CCT GAC GTA GTC GTC AAT GAG TCC TGC TGG AGC GAC CTC GAA    539
   Arg Gly Pro Asp Val Val Val Asn Glu Ser Cys Trp Ser Asp Leu Glu
           145                 150                 155
   TTC TGC AAG AAA ACC AAG AAC TGG TAC TGC TAC GGC AAG GCC GTG GCG    587
   Phe Cys Lys Lys Thr Lys Asn Trp Tyr Cys Tyr Gly Lys Ala Val Ala
       160                 165                 170
   GAG CAG GCT GCG TGG GAG GCG GCC AGG AAG CGC GGC ATC GAC CTC GTC    635
   Glu Gln Ala Ala Trp Glu Ala Ala Arg Lys Arg Gly Ile Asp Leu Val
   175                 180                 185                 190
   GTC GTG AAC CCT GTG CTC GTG GTA GGG CCG CTG CTG CAA CCA ACG GTG    683
   Val Val Asn Pro Val Leu Val Val Gly Pro Leu Leu Gln Pro Thr Val
                   195                 200                 205
   AAC GCT AGC GCC GCA CAC ATC CTC AAG TAC CTC GAC GGC TCG GCC AAG    731
   Asn Ala Ser Ala Ala His Ile Leu Lys Tyr Leu Asp Gly Ser Ala Lys
               210                 215                 220
   AAG TAC GCC AAC GCT GTG CAG TCA TAC GTA GAC GTG CGT GAC GTA GCC    779
   Lys Tyr Ala Asn Ala Val Gln Ser Tyr Val Asp Val Arg Asp Val Ala
           225                 230                 235
   GGC GCG CAC ATC CGG GTG TTC GAG GCG CCT GAG GCG TCG GGC CGG TAC      827
   Gly Ala His Ile Arg Val Phe Glu Ala Pro Glu Ala Ser Gly Arg Tyr
       240                 245                 250
   CTC TGC GCC GAG CGC GTG CTG CAC CGT GGG GAC GTT GTC CAA ATC CTC      875
   Leu Cys Ala Glu Arg Val Leu His Arg Gly Asp Val Val Gln Ile Leu
   255                 260                 265                 270
   AGC AAA CTC TTG CCT GAG TAC CCT GTG CCA ACA AGG TGC TCT GAT GAA      923
   Ser Lys Leu Leu Pro Glu Tyr Pro Val Pro Thr Arg Cys Ser Asp Glu
                   275                 280                 285
   GTG AAC CCA CGG AAG CAG CCT TAT AAG ATG TCC AAC CAG AAG CTG CAG      971
   Val Asn Pro Arg Lys Gln Pro Tyr Lys Met Ser Asn Gln Lys Leu Gln
               290                 295                 300
   GAT CTT GGC CTC CAG TTC ACT CCT GTG AAC GAC TCT CTG TAT GAG ACC     1019
   Asp Leu Gly Leu Gln Phe Thr Pro Val Asn Asp Ser Leu Tyr Glu Thr
           305                 310                 315
   GTG AAG AGC CTC CAG GAG AAG GGA CAT CTC CTA GTA CCA AGC AAA CCC     1067
   Val Lys Ser Leu Gln Glu Lys Gly His Leu Leu Val Pro Ser Lys Pro
       320                 325                 330
   GAG GGA TTA AAC GGT GTA ACG GCA TGATACTGCT AAAGAAGCAG CAGAGTTCAC   1121
   Glu Gly Leu Asn Gly Val Thr Ala
   335                 340
   GTGCTCCTGT AACATGGTCA AACATGAGTT GTTTTTCTGT ATAAATTCTA TCCAGTATCG  1181
   TGTTATTTAA GTGAACTAAG AGAACAGAAT ATTGTATCAT CTTCGATGTC CAATACCTGG  1241
   AAGTGATTTG TTTTGCCACC TAAAAAAAAA AA                                1273
(2) INFORMATION POUR LA SEQ ID NO: 6:
   (i) CARACTERISTIQUES DE LA SEQUENCE:
      (A) LONGUEUR: 342 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 6:
   Met Thr Val Val Asp Ala Ala Ala Pro Gln Leu Pro Gly His Gly Gln
   1               5                   10                  15
   Thr Val Cys Val Thr Gly Ala Ala Gly Tyr Ile Ala Ser Gly Leu Val
               20                  25                  30
   Lys Leu Leu Leu Glu Arg Gly Tyr Thr Val Lys Gly Thr Val Arg Asn
           35                  40                  45
   Pro Asp Asp Pro Lys Asn Ala His Leu Lys Ala Leu Asp Gly Ala Thr
       50                  55                  60
   Lys Arg Leu Ile Leu Cys Lys Ala Asp Leu Leu Asp Tyr Asp Ala Ile
   65                  70                  75                  80
   Cys Ala Ala Val Glu Gly Cys His Gly Val Phe His Thr Ala Ser Pro
                   85                  90                  95
   Val Thr Asp Asp Pro Glu Gln Met Val Glu Pro Ala Val Arg Gly Thr
               100                 105                 110
   Glu Tyr Val Ile Asn Ala Ala Ala Asp Ala Gly Thr Val Arg Arg Val
           115                 120                 125
   Val Phe Thr Ser Ser Ile Gly Ala Ile Thr Met Asp Pro Asn Arg Gly
       130                 135                 140
   Pro Asp Val Val Val Asn Glu Ser Cys Trp Ser Asp Leu Glu Phe Cys
   145 150 155 160
   Lys Lys Thr Lys Asn Trp Tyr Cys Tyr Gly Lys Ala Val Ala Glu Gln
   165 170 175
   Ala Ala Trp Glu Ala Ala Arg Lys Arg Gly Ile Asp Leu Val Val Val
   180 185 190
   Asn Pro Val Leu Val Val Gly Pro Leu Leu Gln Pro Thr Val Asn Ala
   195 200 205
   Ser Ala Ala His Ile Leu Lys Tyr Leu Asp Gly Ser Ala Lys Lys Tyr
   210 215 220
   Ala Asn Ala Val Gln Ser Tyr Val Asp Val Arg Asp Val Ala Gly Ala
   225 230 235 240
   His Ile Arg Val Phe Glu Ala Pro Glu Ala Ser Gly Arg Tyr Leu Cys
   245 250 255
   Ala Glu Arg Val Leu His Arg Gly Asp Val Val Gln Ile Leu Ser Lys
   260 265 270
   Leu Leu Pro Glu Tyr Pro Val Pro Thr Arg Cys Ser Asp Glu Val Asn
   275 280 285
   Pro Arg Lys Gln Pro Tyr Lys Met Ser Asn Gln Lys Leu Gln Asp Leu
   290 295 300
   Gly Leu Gln Phe Thr Pro Val Asn Asp Ser Leu Tyr Glu Thr Val Lys
   305 310 315 320
   Ser Leu Gln Glu Lys Gly His Leu Leu Val Pro Ser Lys Pro Glu Gly
   325 330 335
   Leu Asn Gly Val Thr Ala
   340
(2) INFORMATION POUR LA SEQ ID NO: 7:
   (i) CARACTERISTIQUES DE LA SEQUENCE:
      (A) LONGUEUR: 1293 paires de bases
      (B) TYPE: acide nucléique
      (C) NOMBRE DE BRINS: simple
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 95..1108
   (xi) DESCRIPTION DE LA SEQUENCE: SEQ ID NO: 7:
   CCGAGCCTAT TTCTTCCCTA TATCCACTCA TCCTTGTCTT ATATCATCAT CATCATCATC      60
   TACCTAAACC TGAGCTCAAC AGAAAAGTAA TACC ATG CCG TCA GTT TCC GGC         112
                                         Met Pro Ser Val Ser Gly
                                           1               5
   CAA ATC GTT TGT GTT ACT GGC GCC GGA GGT TTC ATC GCC TCT TGG CTC       160
   Gln Ile Val Cys Val Thr Gly Ala Gly Gly Phe Ile Ala Ser Trp Leu
                   10                  15                  20
   GTT AAA ATT CTT CTG GAA AAA GGC TAC ACT GTT AGA GGA ACA GTA CGA       208
   Val Lys Ile Leu Leu Glu Lys Gly Tyr Thr Val Arg Gly Thr Val Arg
               25                  30                  35
   AAT CCA GAT GAT CGA AAA AAT AGT CAT TTG AGG GAG CTT GAA CGA GCA       256
   Asn Pro Asp Asp Arg Lys Asn Ser His Leu Arg Glu Leu Glu Arg Ala
           40                  45                  50
   AAA GAG ACA TTG ACT CTG TGC AGA GCT GAT CTT CTT GAT TTT CAG AGT       304
   Lys Glu Thr Leu Thr Leu Cys Arg Ala Asp Leu Leu Asp Phe Gln Ser
    55                  60                  65                  70
   TTG CGA GAA GCA ATC AGC GGC TGT GAC GGA GTT TTC CAC ACA CGT TCT       352
   Leu Arg Glu Ala Ile Ser Gly Cys Asp Gly Val Phe His Thr Arg Ser
                        75                  80                  85
   CCT GTC ACT GAT GAT CCA GAA CAA ATG GTG GAG CCA GCA GTT ATT GGT       400
   Pro Val Thr Asp Asp Pro Glu Gln Met Val Glu Pro Ala Val Ile Gly
                    90                  95                 100
   ACA AAG AAT GTG ATA ACG GCA GCA GCA GAG GCC AAG GTG CGA CGT GTG       448
   Thr Lys Asn Val Ile Thr Ala Ala Ala Glu Ala Lys Val Arg Arg Val
               105                 110                 115
   GTG TTC ACT TCG TCA ATT GGT GCT GTG TAT ATG GAC CCA AAC AGG GAC       496
   Val Phe Thr Ser Ser Ile Gly Ala Val Tyr Met Asp Pro Asn Arg Asp
       120                 125                 130
   CCT GAT AAG GTT GTC GAC GAG ACT TGT TGG AGT GAT CCT GAC TTC TGC       544
   Pro Asp Lys Val Val Asp Glu Thr Cys Trp Ser Asp Pro Asp Phe Cys
   135                 140                 145                 150
   AAA AAC ACC AAG AAT TGG TAT TGT TAT GGG AAG ATG GTG GCA GAA CAA       592
   Lys Asn Thr Lys Asn Trp Tyr Cys Tyr Gly Lys Met Val Ala Glu Gln
                   155                 160                 165
   GCA GCA TGG GAC GAA GCA AGG GAG AAA GGA GTC GAT TTG GTG GCA ATC       640
   Ala Ala Trp Asp Glu Ala Arg Glu Lys Gly Val Asp Leu Val Ala Ile
               170                 175                 180
   AAC CCA GTG TTG GTG CTT GGA CCA CTG CTC CAA CAG AAT GTG AAT GCC       688
   Asn Pro Val Leu Val Leu Gly Pro Leu Leu Gln Gln Asn Val Asn Ala
           185                 190                 195
   AGT GTT CTT CAC ATC CAC AAG TAC CTA ACT GGC TCT GCT AAA ACA TAT       736
   Ser Val Leu His Ile His Lys Tyr Leu Thr Gly Ser Ala Lys Thr Tyr
       200                 205                 210
   ACG TCC AAT TCA CTT CAG GCA TAT GTT CAT GTT AGG GAT GTG GCT TTA        784
   Thr Ser Asn Ser Leu Gln Ala Tyr Val His Val Arg Asp Val Ala Leu
   215                 220                 225                 230
   CGT CAC ATA CTT GTG TAC GAG ACA CCT TCT GCA TCT GGC CGT TAT CTC        832
   Arg His Ile Leu Val Tyr Glu Thr Pro Ser Ala Ser Gly Arg Tyr Leu
                   235                 240                 245
   TGT GCC GAG AGT GTG CTG CAT CGC TGC GAT GTG GTT GAA ATT CTC GCC        880
   Cys Ala Glu Ser Val Leu His Arg Cys Asp Val Val Glu Ile Leu Ala
               250                 255                 260
   AAA TTC TTC CCG GAG TAT CCT ATC CCC ACC AAG TGT TCA GAT GTG ACG        928
   Lys Phe Phe Pro Glu Tyr Pro Ile Pro Thr Lys Cys Ser Asp Val Thr
           265                 270                 275
   AAG CCA AGG GTA AAA CCG TAC AAA TTC TCA AAC CAA AAG CTA AAG GAT        976
   Lys Pro Arg Val Lys Pro Tyr Lys Phe Ser Asn Gln Lys Leu Lys Asp
       280                 285                 290
   TTG GGT CTG GAG TTT ACA CCA GTA CAA TGC TTA TAT GAA ACG GTG AAG       1024
   Leu Gly Leu Glu Phe Thr Pro Val Gln Cys Leu Tyr Glu Thr Val Lys
   295                 300                 305                 310
   AGT CTA CAA GAG AAA GGT CAC CTT CCA ATT CCT ACT CAA AAG GAT GAG       1072
   Ser Leu Gln Glu Lys Gly His Leu Pro Ile Pro Thr Gln Lys Asp Glu
                   315                 320                 325
   ATT ATT CGA ATT CAG TCT GAG AAA TTC AGA AGC TCT TAGCATGTAT            1118
   Ile Ile Arg Ile Gln Ser Glu Lys Phe Arg Ser Ser
               330                 335
   TGAGGAAAAG GGATCAATGG TTAAAGTTGA CCATGGCGTT GTCCCTTTAT GTACCAAGAC     1178
   CAAATGCACC TAGAAATTTA CTTGTCTACT CTGTTGTACT TTTACTTGTC ATGGAAATGT     1238
   TTTTAGTGTT TTCATTGTTA TGAGATATAT TTTGGTGTAA AAAAAAAAAA AAAAA          1293
(2) INFORMATION POUR LA SEQ ID NO: 8:
   (i) CARACTERISTIQUES DE LA SEQUENCE:
      (A) LONGUEUR: 338 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 8:
   Met Pro Ser Val Ser Gly Gln Ile Val Cys Val Thr Gly Ala Gly Gly
   1               5                   10                  15
   Phe Ile Ala Ser Trp Leu Val Lys Ile Leu Leu Glu Lys Gly Tyr Thr
               20                  25                  30
   Val Arg Gly Thr Val Arg Asn Pro Asp Asp Arg Lys Asn Ser His Leu
           35                  40                  45
   Arg Glu Leu Glu Arg Ala Lys Glu Thr Leu Thr Leu Cys Arg Ala Asp
       50                  55                  60
   Leu Leu Asp Phe Gln Ser Leu Arg Glu Ala Ile Ser Gly Cys Asp Gly
   65                  70                  75                  80
   Val Phe His Thr Arg Ser Pro Val Thr Asp Asp Pro Glu Gln Met Val
                   85                  90                  95
   Glu Pro Ala Val Ile Gly Thr Lys Asn Val Ile Thr Ala Ala Ala Glu
               100                 105                 110
   Ala Lys Val Arg Arg Val Val Phe Thr Ser Ser Ile Gly Ala Val Tyr
           115                 120                 125
   Met Asp Pro Asn Arg Asp Pro Asp Lys Val Val Asp Glu Thr Cys Trp
       130                 135                 140
   Ser Asp Pro Asp Phe Cys Lys Asn Thr Lys Asn Trp Tyr Cys Tyr Gly
   145 150 155 160
   Lys Met Val Ala Glu Gln Ala Ala Trp Asp Glu Ala Arg Glu Lys Gly
   165 170 175
   Val Asp Leu Val Ala Ile Asn Pro Val Leu Val Leu Gly Pro Leu Leu
   180 185 190
   Gln Gln Asn Val Asn Ala Ser Val Leu His Ile His Lys Tyr Leu Thr
   195 200 205
   Gly Ser Ala Lys Thr Tyr Thr Ser Asn Ser Leu Gln Ala Tyr Val His
   210 215 220
   Val Arg Asp Val Ala Leu Arg His Ile Leu Val Tyr Glu Thr Pro Ser
   225 230 235 240
   Ala Ser Gly Arg Tyr Leu Cys Ala Glu Ser Val Leu His Arg Cys Asp
   245 250 255
   Val Val Glu Ile Leu Ala Lys Phe Phe Pro Glu Tyr Pro Ile Pro Thr
   260 265 270
   Lys Cys Ser Asp Val Thr Lys Pro Arg Val Lys Pro Tyr Lys Phe Ser
   275 280 285
   Asn Gln Lys Leu Lys Asp Leu Gly Leu Glu Phe Thr Pro Val Gln Cys
   290 295 300
   Leu Tyr Glu Thr Val Lys Ser Leu Gln Glu Lys Gly His Leu Pro Ile
   305 310 315 320
   Pro Thr Gln Lys Asp Glu Ile Ile Arg Ile Gln Ser Glu Lys Phe Arg
   325 330 335
   Ser Ser
(2) INFORMATION POUR LA SEQ ID NO: 9:
   (i) CARACTERISTIQUES DE LA SEQUENCE:
      (A) LONGUEUR: 1297 paires de bases
      (B) TYPE: acide nucléique
      (C) NOMBRE DE BRINS: double
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 136..1140
   (xi) DESCRIPTION DE LA SEQUENCE: SEQ ID NO: 9:
   CGGCCGGGAC GACCCGTTCC TCTTCTTCCG GGTCACCGTC ACCATGTTAC ACAACATCTC       60
   CGGCTAAAAA AAAAAGGAAA AAAAGCGCAA CCTCCACCTC CTGAACCCCT CTCCCCCCTC      120
   GCCGGCAATC CCACC ATG CCC GTC GAC GCC CTC CCC GGT TCC GGC CAG ACC      171
                    Met Pro Val Asp Ala Leu Pro Gly Ser Gly Gln Thr
                      1               5                  10
   GTC TGC GTC ACC GGC GCC GGC GGG TTC ATC GCC TCC TGG ATT GTC AAG       219
   Val Cys Val Thr Gly Ala Gly Gly Phe Ile Ala Ser Trp Ile Val Lys
            15                  20                  25
   CTT CTC CTC GAG CGA GGC TAC ACC GTG CGA GGA ACC GTC AGG AAC CCA       267
   Leu Leu Leu Glu Arg Gly Tyr Thr Val Arg Gly Thr Val Arg Asn Pro
        30                  35                  40
   GAC GAC CCG AAG AAT GGT CAT CTG AGA GAT CTG GAA GGA GCC AGC GAG       315
   Asp Asp Pro Lys Asn Gly His Leu Arg Asp Leu Glu Gly Ala Ser Glu
    45                  50                  55                  60
   AGG CTG ACG CTG TAC AAG GGT GAT CTG ATG GAC GAC GGG AGC TTG GAA       363
   Arg Leu Thr Leu Tyr Lys Gly Asp Leu Met Asp Asp Gly Ser Leu Glu
                    65                  70                  75
   GAA GCC ATC AAG GGG TGC GAC GGC GTC GTC CAC ACC GCC TCT CCG GTC 411
   Glu Ala Ile Lys Gly Cys Asp Gly Val Val His Thr Ala Ser Pro Val
   80 85 90
   ACC GAC GAT CCT GAG CAA ATG GTG GAG CCA GCG GTG ATC GGG ACG AAA 459
   Thr Asp Asp Pro Glu Gln Met Val Glu Pro Ala Val Ile Gly Thr Lys
   95 100 105
   AAT GTG ATC GTC GCA GCG GCG GAG GCC AAG GTC CGG CGG GTT GTG TTC 507
   Asn Val Ile Val Ala Ala Ala Glu Ala Lys Val Arg Arg Val Val Phe
   110 115 120
   ACC TCC TCC ATC GGT GCA GTC ACC ATG GAC CCC AAC CGG GCA GAC GTT 555
   Thr Ser Ser Ile Gly Ala Val Thr Met Asp Pro Asn Arg Ala Asp Val
   125 130 135 140
   GTG GTG GAC GAG TCT TGT TGG AGC GAC CTC GAA TTT TGC AAG AGC ACT 603
   Val Val Asp Glu Ser Cys Trp Ser Asp Leu Glu Phe Cys Lys Ser Thr
   145 150 155
   AAG AAC TGG TAT TGC TAC GGC AAG GCA GTG GCG GAG AAG GCC GCT TGG 651
   Lys Asn Trp Tyr Cys Tyr Gly Lys Ala Val Ala Glu Lys Ala Ala Trp
   160 165 170
   CCA GAG GGC AAG GAG AGA GGG GTT GAC CTC GTG GTG ATT AAC CCT GTG 699
   Pro Glu Gly Lys Glu Arg Gly Val Asp Leu Val Val Ile Asn Pro Val
   175 180 185
   CTC GTG CTT GGA CCG CTC CTT CAG TCG ACG ATC AAT GCG AGC ATC ATC 747
   Leu Val Leu Gly Pro Leu Leu Gln Ser Thr Ile Asn Ala Ser Ile Ile
   190 195 200
   CAC ATC CTC AAG TAC TTG ACT GGC TCA GCC AAG ACC TAC GCC AAC TCG 795
   His Ile Leu Lys Tyr Leu Thr Gly Ser Ala Lys Thr Tyr Ala Asn Ser
   205 210 215 220
   GTC CAG GCG TAC GTG CAC GTC AAG GAC GTC GCG CTT GCC CAC GTC CTT      843
   Val Gln Ala Tyr Val His Val Lys Asp Val Ala Leu Ala His Val Leu
                   225                 230                 235
   GTC TTG GAG ACC CCA TCC GCC TCA GGC CGC TAT TTG TGC GCC GAG AGC      891
   Val Leu Glu Thr Pro Ser Ala Ser Gly Arg Tyr Leu Cys Ala Glu Ser
               240                 245                 250
   GTC CTC CAC CGT GGC GAT GTG GTG GAA ATC CTT GCC AAG TTC TTC CCT      939
   Val Leu His Arg Gly Asp Val Val Glu Ile Leu Ala Lys Phe Phe Pro
           255                 260                 265
   GAG TAT AAT GTA CCG ACC AAG TGC TCT GAT GAG GTG AAC CCA AGA GTA      987
   Glu Tyr Asn Val Pro Thr Lys Cys Ser Asp Glu Val Asn Pro Arg Val
       270                 275                 280
   AAA CCA TAC AAG TTC TCC AAC CAG AAG CTG AGA GAC TTG GGG CTC GAG     1035
   Lys Pro Tyr Lys Phe Ser Asn Gln Lys Leu Arg Asp Leu Gly Leu Glu
   285                 290                 295                 300
   TTC ACC CCG GTG AAG CAG TGC CTG TAC GAA ACT GTC AAG AGC TTG CAG     1083
   Phe Thr Pro Val Lys Gln Cys Leu Tyr Glu Thr Val Lys Ser Leu Gln
                   305                 310                 315
   GAG AAA GGC CAC CTA CCA GTC CCC TCC CCG CCG GAA GAT TCG GTG CGT     1131
   Glu Lys Gly His Leu Pro Val Pro Ser Pro Pro Glu Asp Ser Val Arg
               320                 325                 330
   ATT CAG GGA TGATCTTAGA TCCATCACGG TGCGCATTTG TAATCCGGAG             1180
   Ile Gln Gly
           335
   AAATGAGAGA AACATGTGGG AATTTGTTTG TACTTTTCTA AGTCAAACCT GGAGATACCA   1240
   ACCCTGAGTT CTGCATTGGA ATGGAAGTTG TCAATTGTTC CAAAAAAAAA AAAAAAA      1297
(2) INFORMATION POUH LA SEQ ID NO: 10:
   (i) CARACTERISTIQUES DE LA SEQUENCE:
      (A) LONGUEUR: 335 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 10: